# EUROPEAN PATENT APPLICATION

(11) **EP 4 817 561 A2**
(43) Date of publication of application: **07.10.2026**
(21) Application number: 26199826.4
(22) Date of filing: 30.09.2020
(51) Int. Cl.: A61F 2/40

(54) **REVERSE SHOULDER SYSTEMS**

(30) Priority: 01.10.2019 US 201962908921 P
(62) Document number(s) of the earlier application(s) in accordance with Art. 76 EPC: 25203423.6 / 4 647 044; 20796968.4 / 4 037 615
(71) Applicant: Howmedica Osteonics Corp., Mahwah, NJ 07430 (US)
(72) Inventor: WOLFE, Alexander Paul, BLOOMINGTON, 55437 (US); STUMP, David R, BLOOMINGTON, 55437 (US); KNOX, Kevin P., BLOOMINGTON, 55437 (US); GABORIT, Vincent, BLOOMINGTON, 55437 (US); DASSONVILLE, Benjamin, BLOOMINGTON, 55437 (US); DERANSART, Pierric, BLOOMINGTON, 55437 (US)
(74) Representative: Lavoix

(57) **Abstract**

The present disclosure are directed toward an articular assembly configured to be coupled with a bone anchor. The articular assembly may include an articular body having a first end and a second end with an articular surface disposed on or adjacent to the first end. The articular body may include a bone anchor interface disposed between the first end and the second end of the articular body. The bone anchor interface may include a channel formed in a circumferential surface of the articular body, a locking member disposed in the channel, and/or a deflectable portion disposed between the locking member and the second end of the articular body.

## Description

### INCORPORATION BY REFERENCE TO ANY PRIORITY APPLICATIONS

This application claims priority benefit of U.S. Provisional Application No. 62/908921, filed October 1, 2019, which is hereby incorporated by reference in its entirety herein.

### BACKGROUND

### Field

The present application relates to apparatuses and methods for reverse shoulder prostheses.

### Description of the Related Art

Arthroplasty is the standard of care for the treatment of shoulder joint arthritis. A typical anatomical shoulder joint replacement attempts to mimic anatomic conditions. A metallic humeral stem and a humeral head replacement are attached to the humerus of the arm and replace the humeral side of the arthritic shoulder joint. Such humeral head replacement can articulate with the native glenoid socket or with an opposing glenoid resurfacing device.

For more severe cases, a reverse reconstruction can be employed. In a reverse reconstruction the kinematics of the shoulder joint are reversed by securing a spherical device (sometimes called a glenoid sphere) to the glenoid and implanting a humeral implant with a cavity capable of receiving the glenoid sphere.

A challenge in reverse shoulder assemblies is that the articular body is usually made of a low friction polymer and the humeral anchor of a metal. Metals and polymers used in reverse articular bodies can wear at different rates. Also, there a risk of unintended disconnection between the articular body and the anchor in a typical assembly. Accordingly, there remains a continuing need for improved shoulder prosthesis components and assemblies.

### SUMMARY

Improvements are needed in connection components that are used to connect multiple portions of shoulder joint assemblies together, for example to prevent relative motion between components of the joint assembly.

Certain aspects of the present disclosure are directed to an articular component configured to be coupled with a bone anchor. The articular component may include an articular body having a first end and a second end. An articular surface may be disposed on or adjacent to the first end. A bone anchor interface may be disposed between the first end and the second end of the articular body. The bone anchor interface may include a locking member configured to secure the articular component to the bone anchor and/or a deflectable portion disposed at the second end of the articular body. The deflectable portion may be configured to be circumferentially deflected by a surface of a humeral anchor to provide a load directed toward the first end of the articular body from a second end of the articular body when deflected.

The articular body may include a transverse surface configured to overlay a rim of the bone anchor when the locking member is engaged with the bone anchor.

The bone anchor interface may include a channel formed in a circumferential surface of the articular body. The locking member may be disposed in the channel.

The deflectable portion may include at least two sections cantilevered from a central portion of the articular body to the second end of the articular body. The deflectable portion may include a compression slot disposed between each of the at least two sections. The deflectable portion may a tapered surface disposed on an outer circumference thereof. The deflectable portion may be disposed between the locking member and the second end of the articular body.

The articular component may include a rotation control zone disposed at a periphery of the articular body between the first end and the second end. The rotation control zone may include a projection disposed in a first direction and a recess disposed in a second direction. The first direction may be transverse to the second direction. The projection may be a first projection and may further include a second projection disposed opposite the first projection. The recess may be a first recess and further include a second recess disposed opposite the first recess.

Any of the articular components described herein may be included in a kit. The kit including a bone anchor having a bone anchor recess formed therein. The bone anchor recess may extend from a first end. A bone engagement outer surface may extend from the first end to a second end opposite the first end. The bone anchor recess may include a first peripheral portion adjacent to the first end configured to engage the locking member of the articular component and a second peripheral portion between the first peripheral portion and the second end. The second peripheral portion may be configured to engage the deflectable portion. Optionally, the kit may include a tray or spacer having a first end and a second end configured to engage the bone anchor recess of the bone anchor at least at the second peripheral portion. The first end of the tray may include a tray recess formed therein. The tray recess may include a first peripheral portion adjacent to the first end configured to engage the locking member of the articular component and a second peripheral portion between the first peripheral portion and the second end. The second peripheral portion configured to engage the deflectable portion.

Certain aspects of the present disclosure are directed toward a humeral assembly. The humeral assembly may include a humeral anchor (e.g., stemmed or stemless) and an articular assembly. The humeral anchor may be configured to be anchored in a bone. The humeral anchor may include a first end, a second end, and a recess extending between the first end and the second end. The recess may be accessible from the first end of the humeral anchor and include a first peripheral portion adjacent to the first end and a second peripheral portion between the first peripheral portion and the second end of the humeral anchor. The articular assembly may be configured to be inserted into the recess to be secured to the humeral anchor therein. The articular assembly may include an articular body having an articular surface disposed on or adjacent to a first end thereof and a humeral anchor interface disposed between the first end and a second end of the articular body. The humeral anchor interface may include a channel formed in a circumferential surface of the articular body, a locking member disposed in the channel, and/or a deflectable projection disposed between the locking member (e.g., locking ring or C-ring) and the second end of the articular body.

The deflectable projection may be configured to be positioned in the second peripheral portion of the recess, and when so positioned, to be circumferentially deflected by a surface of the second peripheral portion and/or to provide a load between the locking member and a surface of the first peripheral portion. When the humeral anchor and the articular assembly are coupled together, the deflectable projection is configured to engage a surface surrounding the second peripheral portion before the locking member engages the first peripheral portion of the recess of the humeral anchor.

Certain aspects of the present disclosure are directed toward an articular assembly configured to be coupled with a bone anchor. The articular assembly may include an articular body having a first end and a second end with an articular surface disposed on or adjacent to the first end. The articular body may include a bone anchor interface disposed between the first end and the second end of the articular body. The bone anchor interface may include a channel formed in a circumferential surface of the articular body, a locking member disposed in the channel, and/or a deflectable portion disposed between the locking member and the second end of the articular body. The deflectable portion may be configured to be circumferentially deflected by a surface of a humeral anchor and/or to provide a load directed toward the first end of the articular body from a second end of the articular body, for example when frusto-conically deflected.

The articular body may include a transverse surface disposed between the channel and the first end of the articular body. The transverse surface may be configured to overlay a rim of a humeral anchor when the locking member is engaged with the humeral anchor.

The articular body may include a rotation control zone disposed at a periphery of the articular body between the first end and the second end. The rotation control zone includes at least one projection disposed in a first direction and at least one recess disposed in a second direction. The first direction may be transverse to the second direction.

The deflectable portion may include a tapered surface disposed on an outer circumference thereof. In some configurations, the deflectable projection includes a blind hole along a centerline of the articular body. In some configurations, the deflectable portion includes at least two sections, for example four sections, cantilevered from a central portion of the articular body to the second end of the articular body. The deflectable portion may also include a compression slot disposed between each of the at least two sections.

Any of the articular assemblies described herein may form part of a kit. The kit may include a bone anchor having a bone anchor recess formed therein. The bone anchor recess extends from a first end. The bone engagement outer surface extends from the first end to a second end opposite the first end. The bone anchor recess may have a first peripheral portion adjacent to the first end configured to engage the locking member of the articular assembly and a second peripheral portion between the first peripheral portion and the second end. The second peripheral portion may be configured to engage the deflectable portion of the articular assembly.

In some embodiments, the kit may include a tray having a first end and a second end configured to engage the bone anchor recess of the bone anchor at least at the second peripheral portion. The first end of the tray may include a tray recess formed therein. The tray recess may include a first peripheral portion adjacent to the first end configured to engage the locking member of the articular assembly and a second peripheral portion between the first peripheral portion and the second end. The second peripheral portion may be configured to engage the deflectable portion.

In use, a bone anchor may be positioned in an end of a long bone of a patient. An articular assembly may be rotationally aligning with the bone anchor by rotationally aligning a first rotational alignment feature (e.g., a projection or a concavity) of an articular assembly with a second rotational alignment feature (e.g., the negative of the first rotational alignment feature) disposed in a recess. The recess may be formed in the bone anchor or in a tray coupled with the bone anchor disposed at the end of the long bone. The articular assembly may be advanced into the bone anchor until a counter-load projection is disposed within a tapered surface of the recess. For example, the articular assembly may be advanced until tapered outer surfaces of the counter-load projection engages with a tapered surface of the recess to move sections of the counter-load projection toward each other across compression slots thereof. The articular assembly may be further advanced until a locking member of the articular assembly is deflected within a channel formed in a central portion of the articular body. The articular assembly may be further advanced until the locking member of the articular assembly is aligned with a channel disposed about the recess in the bone anchor to permit the locking member to span a gap between the channel in the articular body and the channel disposed about the recess. When the counter-load projection is deflected by the tapered surface of the recess, the counter-load projection reduces, minimizes or eliminates motion of the articular assembly relative to the bone anchor after the locking member spans the gap between the channel in the articular body and the channel disposed about the recess.

Certain aspects of this disclosure are directed toward a shoulder joint prosthesis assembly including a bone anchor and an articular assembly. The bone anchor may include a metallic body, while the articular assembly includes a polymeric body. The bone anchor may include a body that has a bone engaging side to be placed against bone and an assembly side opposite the bone engaging side. The assembly side may include a recess disposed about a mounting area. The mounting area may include a channel disposed peripherally about the mounting area. The channel provides a first retention surface. The articular assembly has a body portion and a locking member. The body extends along a central insertion axis between a first portion configured to be inserted into the mounting area and a second portion opposite the first portion. The second portion includes an articular surface.

In assemblies with a locking member, the locking member may include an arcuate member disposed about the first portion of the articular body. The arcuate member has a second retention surface. The second retention surface may be disposed at an angle, for example an acute angle, to a plane disposed perpendicular to the central insertion axis. A first portion of the second retention surface may be disposed farther from the articular surface in the direction of the central insertion axis than the first retention surface when the articular assembly is engaged with the bone anchor. A second portion of the second retention surface may be disposed closer to the articular surface in the direction of the central insertion axis than is the first retention surface when the articular assembly is engaged with the bone anchor. The first retention surface may be disposed between inner and outer ends of the second retention surface when the locking member engages the channel of the bone anchor.

The locking member may also include a resilient body. A first end of the resilient body may extend from the first end of the arcuate member. A second end of the resilient body may interface with or engage the polymeric body. The first portion of the body portion may include a recess configured to receive the resilient body in only one position. The resilient body may be configured to center the arcuate member relative to the central insertion axis. For example, the resilient body stores strain energy upon application of a deflection force to deflect the first end of the locking member away from a centered position and releases the strain energy to return the locking member toward the centered position upon removal of the deflection force.

Certain aspects of the disclosure are directed toward a shoulder joint prosthesis assembly including a bone anchor and an articular assembly. The bone anchor may include a metallic body, while the articular assembly includes a polymeric body. The bone anchor may include a bone engaging side to be placed against bone and an assembly side opposite the bone engaging side. The assembly side may include a recess disposed about a mounting area. The mounting area may include a channel disposed peripherally about the mounting area. The channel includes a first retention surface. The articular assembly may include a body portion and a locking member. The body portion extends along a central insertion axis between a first portion configured to be inserted into the mounting area and a second portion opposite the first portion. The second portion includes an articular surface.

Certain aspects of this disclosure are directed toward an articular portion including a body portion, e.g. polymeric body portion, and a locking member. The body portion extends along a central insertion axis between a first portion configured to be inserted into a bone anchor and a second portion opposite the first portion. The second portion includes an articular surface. The locking member may include and an arcuate member disposed about the first portion and a locator body having a first end coupled to the arcuate member and a second end coupled to the polymeric body. The locator body is configured to be received in a recess of the body portion in a pre-defined orientation and/or position.

Any feature, structure, or step disclosed herein can be replaced with or combined with any other feature, structure, or step disclosed herein, or omitted. Further, for purposes of summarizing the disclosure, certain aspects, advantages, and features of the inventions have been described herein. It is to be understood that not necessarily any or all such advantages are achieved in accordance with any particular embodiment of the inventions disclosed herein. No individual aspects of this disclosure are essential or indispensable.

### BRIEF DESCRIPTION OF THE DRAWINGS

These and other features, aspects and advantages are described below with reference to the drawings, which are intended for illustrative purposes and should in no way be interpreted as limiting the scope of the embodiments. Furthermore, various features of different disclosed embodiments can be combined to form additional embodiments, which are part of this disclosure. In the drawings, like reference characters denote corresponding features consistently throughout similar embodiments. The following is a brief description of each of the drawings.
FIG. 1 illustrates a reverse total shoulder arthroplasty system in a shoulder joint, the system including a humeral stem anchor.
FIG. 2 illustrates a schematic diagram of a shoulder arthroplasty system including an arthroplasty kit that can be used in performing anatomic or reverse arthroplasty, in converting from one of anatomic to reverse, or reverse to anatomic arthroplasty.
FIG. 3A illustrates an example of an articular assembly.
FIG. 3B illustrates a cross-sectional view of the articular assembly of FIG. 3A, the section being taken along line 3B-3B.
FIG. 3C illustrates a bottom perspective view of the articular assembly of FIG. 3A.
FIG. 3D illustrates an articular assembly with an asymmetric articular body configuration with an angled medial edge or side.
FIG. 3E illustrates a cross-sectional view of the articular assembly of FIG. 3D, the section being taken along line 3E-3E.
FIG. 3F illustrates an articular assembly with an alternative humeral anchor interface.
FIG. 3G illustrates a cross-sectional view of the articular assembly of FIG. 3E, the section plane extending in a medial-lateral direction through a center of the assembly.
FIG. 4A illustrates a perspective view of a humeral anchor and an articular assembly from the system of FIG. 2 prior to inserting the articular assembly and showing engagement features of these components.
FIG. 4B illustrates the humeral anchor and the articular assembly of FIG. 4A after the articular assembly has been inserted.
FIG. 4C illustrates a cross-sectional view of the humeral assembly of FIG. 4B, the section being taken transverse to the direction of insertion of the reverse articular assembly at the section plane 4C-4C.
FIG. 4D illustrates a top view of the assembled humeral assembly of FIG. 4B.
FIG. 4E illustrates cross-sectional view of a partial assembly of the humeral anchor and the articular assembly of FIG. 4D, the section taken along line 4E,4F-4E,4F.
FIG. 4F illustrates a cross-sectional view of the humeral assembly of FIG. 4D, the section taken along line 4E,4F-4E,4F.
FIG. 5A illustrates a humeral assembly including a stemmed humeral anchor, a tray, and an articular assembly.
FIG. 5B illustrates the tray of the humeral assembly of FIG. 5A.
FIG. 6A illustrates an example of an articular assembly.
FIG. 6B illustrates a bottom view of the articular assembly of FIG. 6A.
FIG. 6C illustrates a locking member of the articular assembly of FIG. 6A.
FIG. 6D illustrates a perspective view of a humeral anchor and the articular assembly of FIG. 6A prior to inserting the articular assembly and showing engagement features of these components.
FIG. 6E illustrates a partial, enlarged cross-section of the connection between the humeral anchor and the articular assembly shown in FIG. 6D.
FIG. 6F illustrates a partial, enlarged cross-section of the connection between the humeral anchor and the articular assembly shown in FIG. 6D.
FIG. 6G illustrates a partial, enlarged cross-section of the connection between the humeral anchor and the articular assembly shown in FIG. 6D.
FIG. 7 illustrates another example of the articular assembly.
FIG. 8A illustrates another example of the locking member.
FIG. 8B illustrates a variation of the locking member shown in FIG. 8A.
FIG. 9A illustrates another example of an articular assembly and a fracture stem.
FIG. 9B illustrates a bottom perspective view of the articular assembly of FIG. 9A.
FIG. 10A illustrates another example of an articular assembly.
FIG. 10B illustrates a partial cross-section of the articular assembly of FIG. 10A coupled to an anchor.
FIG. 11 illustrates another example of an articular assembly.
FIG. 12 illustrates another example of an articular assembly.

### DETAILED DESCRIPTION

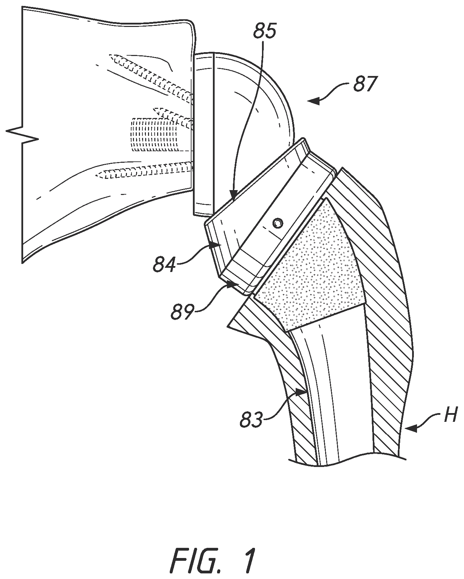

This application is directed in various examples to novel and inventive shoulder implants. The shoulder implants can be part of hemi- and total shoulder joint arthroplasty systems. FIG. 1 shows a reverse approach in which the humerus H is fitted with an articular body 84 having a concave articular surface 85. The glenoid region of the scapula is fitted with a spherical articular body, commonly called a glenosphere 87 (sometimes called a glenoid sphere). In this case, the concave articular surface 85 is placed on the humerus articulates of the glenosphere 87, which is fixed relative to the scapula. The reverse articular body 84 is mounted to a tray 89 that is disposed between the reverse humeral articular body 84 and a stem anchor 83 that is surgically implanted in the humerus *H.* The humerus *H* is prepared by providing access to the medullary canal of the humerus *H*.

### I. SYSTEMS AND KITS WITH SHARED IMPLANT COMPONENTS

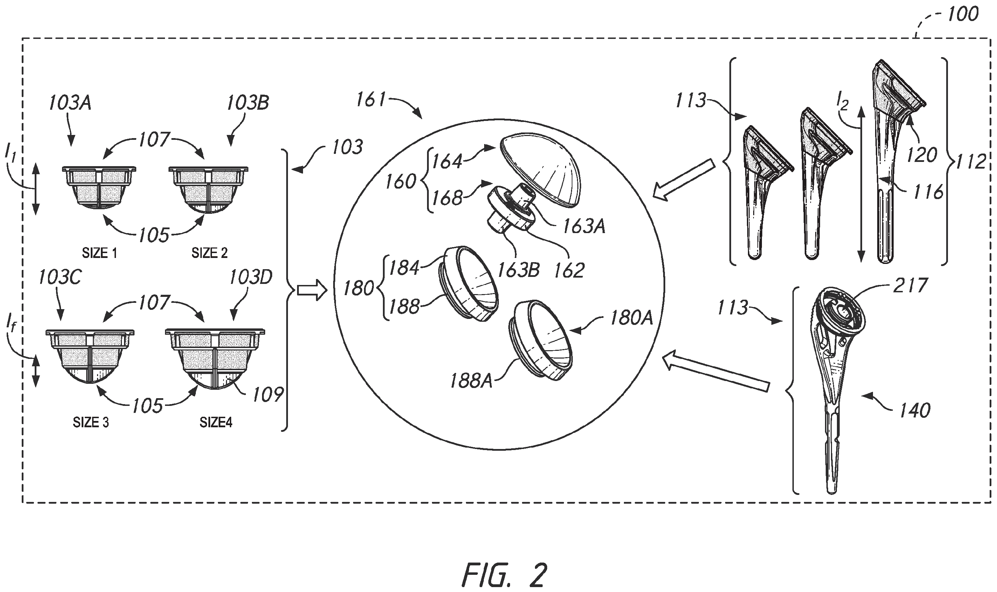

FIG. 2 is a schematic diagram of a total arthroplasty system comprising an arthroplasty kit 100 that can be used to perform anatomic or reverse arthroplasty, or to convert from one of anatomic to reverse or reverse to anatomic arthroplasty, according to various embodiments. The kit 100 can comprise one or a plurality of stemless humeral anchors 103, one or a plurality of stemmed humeral anchors 113, and one or a plurality of articular components 161. The stemless humeral anchors 103 can have a tapered profile in which a distal portion 105 and a proximal portion 107 of the anchor 104. The distal portion 105 of the anchors 103 shown in FIG. 2 can have one or a plurality of fins 109 extending distally. The fins 109 can be configured to secure the anchors 103 into the humerus.

As shown in FIG. 2, the stemless anchors 103 can be provided in a plurality of sizes to accommodate patients of different sizes, different degrees of bone damage to the humerus, etc. In some embodiments, the lateral size of the stemless anchors 103 may vary so as to fit within different-sized resections of the humerus. For example, the kit 100 can comprise a plurality of stemless anchors 103A, 103B, 103C, 103D ... 103*n*, with *n* being the number of different sizes. Although four sizes are illustrated in FIG. 2 (*e.g., n* = 4, with anchors 103A-103D), in other embodiments, the kit can include any suitable number of anchors. In some embodiments, a length *l₁* of the stemless anchors 103A-103D may also vary so as to extend into the humerus by a depth that the clinician selects based on the particular patient being treated. Furthermore, the anchors 103A-103D can have different fin lengths *l_{f}* of the fins 109 to accommodate different sizes of the humerus.

In various embodiments, the fin lengths *l_{f}* of the anchors 103A-103D can differ substantially so as to beneficially provide a wide range of anchor strengths to the humerus and accommodate patients with different levels of bone damage. In the arrangement of FIG. 2, for example, the first anchor 103A can have the shortest overall length *l₁* and the shortest overall fin length *l_{f}*. The fourth anchor 103D can have the longest overall length *l₁* and the longest overall fin length *l_{f}.* In various embodiments, a ratio of an overall length *l₁* of one anchor 103 (for example, the largest anchor 103) to an overall length *l₁* of another anchor 103 (for example, the smallest anchor 103) in the kit 100 can be in a range of 1.15 to 2.5, in a range of 1.18 to 2.5, in a range of 1.2 to 2.5, in a range of 1.2 to 2, in a range of 1.2 to 1.8, in a range of 1.2 to 1.6, in a range of 1.3 to 1.6, or in a range of 1.25 to 1.4.

The kit 100 can also include one or a plurality of stemmed humeral anchors 113. The kit 100 can include one or more humeral stem anchors 112, each of which includes a proximal metaphysis portion 120 and an elongate diaphysis portion 116 extending therefrom. The diaphysis portion 116 is sometimes referred to herein as a stem or stem portion. In some embodiments, the kit 100 can also include a trauma or fracture stem anchor 140, which can be used in patients that have experienced a fracture of the humerus H. The stemmed humeral anchors 113 may be used in patients in which stemless anchors 103 may not be adequately secured to the humerus, for example, in patients that have experienced severe bone loss. The trauma or fracture stem may be used where the humerus has fractured into one or more pieces. As with the stemless anchors 103, the kit 100 can include humeral stem anchors 113 (sometimes referred to herein as a stemmed anchor) having a plurality of different sizes, *e.g.,* different lateral sizes and/or different lengths *l₂.* For example, as shown in FIG. 2, the stemmed humeral anchors 113 can have respective lengths *l₂* that are longer than the lengths *l₁* of the stemless anchors 103. Beneficially, the inclusion of differently-sized stemmed anchors 113 in the kit 100 can enable the clinician to select the appropriate size for a particular patient to ensure a secure implant of the anchor 113 into the patient, in view of the patient's bone size and health. In various embodiments, the lengths *l₂* of the stemmed humeral anchors can be in a range of 55 mm to 175 mm. By contrast, the shorter lengths *l₁* of the stemless humeral anchors 103 can be in a range of 16 mm to 28 mm. In various embodiments, stemmed humeral anchors 113, 140 can be configured to reach into the intramedullary canal of the humerus H for additional anchorage.

Beneficially, the kit 100 can comprise one or a plurality of shared humeral components that be used with either the stemless humeral implants 103 or the stemmed humeral implants 113, depending on which implant 103 or 113 would be more appropriate for a particular patient's humeral anatomy. For example, the shared humeral components of the kit 100 can comprise a plurality of articular components or assemblies 161 that can be used in conjunction with either the stemless implants 103 or the stemmed implants 113. As explained herein, both the stemless humeral anchors 103 and the stemmed humeral anchors 113 can include shared engagement features that can be used with the same set of tools and/or articular components. For example, as described herein, the stemless anchors 103 and stemmed anchors 113 can include convex and concave locking features configured to engage with the same set of articular components.

For example, the kit 100 can include an anatomic articular component 160 configured to mechanically couple to both the stemless humeral implants 103 and the stemmed humeral implants 113. The clinician may select the anatomic articular component 160 for procedures in which an anatomic reconstruction is suitable. The anatomic articular component 160 can comprise a coupler 168 and an articular body 164 (anatomical) configured to mechanically engage the coupler 168. As shown in FIG. 2, the articular body 164 for the anatomic articular component 160 can comprise a rounded, convex surface configured to engage a glenoid surface of the patient. The coupler 168 can serve to mechanically connect the anatomical articular body 164 (*e.g.,* a rounded or essentially spherical surface) to either a stemless humeral implant 103 or a stemmed humeral implant 113, depending on the patient's humeral bone structure. The articular body 164 and the coupler 168 can comprise a metal, such as cobalt, chrome, or titanium. In some embodiments, the articular body comprises a pyrocarbon layer on at least the articular surface. In various embodiments, the kit 100 can include anatomic articular components 160 having a plurality of sizes.

The kit 100 can also include a reverse articular component 180 configured to mechanically couple to both the stemless humeral implants 103 and the stemmed humeral implants 113. The clinician may select the reverse articular component 180 for procedures in which a reverse anatomic reconstruction is suitable. The reverse articular component 180 can comprise a reverse articular body 184 and a locking device 188 configured to secure the reverse articular component 180 to a stemless humeral implant 103 or a stemmed humeral implant 113, depending on the clinician's recommendation during the procedure. As shown, the reverse articular body 184 can comprise a rounded concave surface (e.g., essentially spherical) configured to engage with a glenosphere connected to the glenoid of the patient (not shown but in some cases combined with the kit into a larger surgical kit). In addition, in some embodiments, the kit 100 can include a wear resistant reverse articular component 180A, which may be generally similar to the reverse articular component 180 but may further be formed to include vitamin E to promote long-term compatibility with the patient's bone structure. The reverse components 180, 180A can comprise a polymer, including, for example, ultra-high molecular weight polyethylene. In various embodiments, the kit 100 can include reverse articular components 180, 180A having a plurality of sizes.

During an arthroplasty procedure, the clinician may inspect the bone structure of the humerus and/or the scapula to determine whether the anatomy is suitable for a stemless or stemmed humeral anchor, and whether the anatomy is suitable for an anatomical or reverse anatomical reconstruction. Beneficially, the kit 100 shown in FIG. 2 can provide the clinician with a total arthroplasty system including components that are compatible with stemless or stemmed anchors, and with anatomical or reverse anatomical constructions. For example, during a procedure, the clinician may observe that the patient has sufficient humeral bone structure so that a stemless anchor 103 may be used to reduce the damage to the patient's anatomy. The clinician may also elect whether to proceed with an anatomical reconstruction or a reverse construction, and can accordingly select either the anatomical articular component 160 or the reverse articular component 180, 180A.

Similarly, if during a shoulder arthroplasty procedure, the clinician determines that the patient's bone structure is damaged or otherwise more suited to a stemmed anchor 113, then the clinician can select an appropriately sized stemmed anchor 113. The clinician can further select whether to proceed with an anatomical reconstruction or a reverse construction, and can accordingly select either the anatomical articular component 160 or the reverse articular component 180, 180A. Beneficially, the kit 100 of FIG. 2 includes interchangeable or interoperable components that can be used in stemmed or stemless anchors, and with anatomical or reverse anatomical reconstructions. Because the shared humeral articular components 161 (*e.g*., anatomical or reverse anatomical articular bodies) can be used with either the stemless or stemmed anchors 103, 113, the clinician can make, or change, reconstruction decisions during surgery. The kit 100 can accordingly enable the clinician to quickly determine the reconstruction procedure most suitable for a patient and can provide the clinician with the components to be used for that reconstruction procedure.

As explained above, for humeral fractures, the kit 100 can also include one or more trauma stems 140. Beneficially, the trauma stem(s) 140 can include engagement features generally similar to or the same as the engagement features in the stemless anchors 103 and humeral stem anchors 112, such that the stemless anchors 103, the humeral stem anchors 112, and the trauma stem(s) 140 can be used with a common set of shared articular components 161 and tools. Beneficially, therefore, the kit 100 can provide a shared set of implantation tools and a shared set of articular components 161 that can be used with either stemless or stemmed humeral anchors 103, 113, and that can be used for anatomical or reverse anatomical reconstructions.

In some embodiments, the coupler 168 can comprise a proximal extension 163A configured to connect to the articular body 164 and a distal extension 163B. The distal extension 163B for the fracture stem 140 can be received within a recess 217 of the fracture stem 140 for anatomical reconstructions. The disc or middle portion 162 disposed between the proximal extension 163A and the distal extension 163B can be eliminated since the recess 217 is elevated toward the resection plane. In a modified embodiment, the recess 217 is recessed from (e.g., extends distally from) a distal end of a second recess. In those embodiments, the disc or middle portion 162 provides a spacer function in use in the trauma stem 140. Additional details of trauma stems may be found throughout International Application No. PCT/US2015/065126, filed December 15, 2015, the entire contents of which are hereby incorporated by reference herein in their entirety and for all purposes.

The final implant can take any suitable configuration, such as any that are described in Application No. PCT/US2019/054007, titled "SHOULDER PROSTHESIS COMPONENTS AND ASSEMBLIES," and Application No. PCT/US2019/054023, titled "MODULAR HUMERAL HEAD," which are filed on the same day as the present application and are Attorney Reference Nos. TRNXSH.104WO2 and TRNXSH.105WO, respectively. The final implant can take any configuration as disclosed in Application No. 62/908,725, titled "SHOULDER PROSTHESIS COMPONENTS AND ASSEMBLIES," filed on the same day as the present application and are Attorney Reference No. TRNXSH.104PR2. The entire contents of each of the applications listed in this paragraph are hereby incorporated by reference herein in their entirety and for all purposes.

### II. EXAMPLES OF COMPONENTS OF THE HUMERAL ASSEMBLY

As noted above, this application discloses some kits and systems that provide shared components and that may include multiple types of articular assemblies. For example, a humeral assembly may include an articular portion and an anchor.

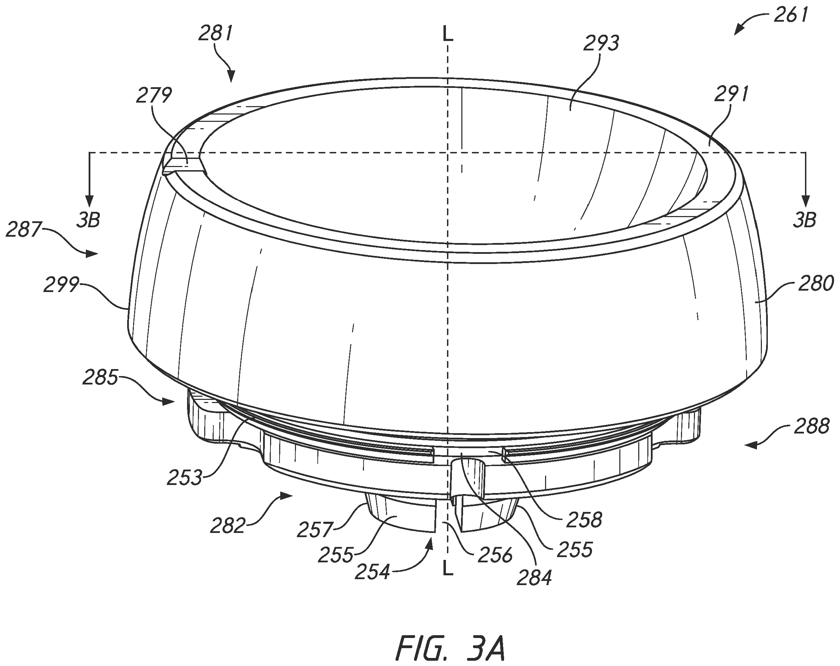

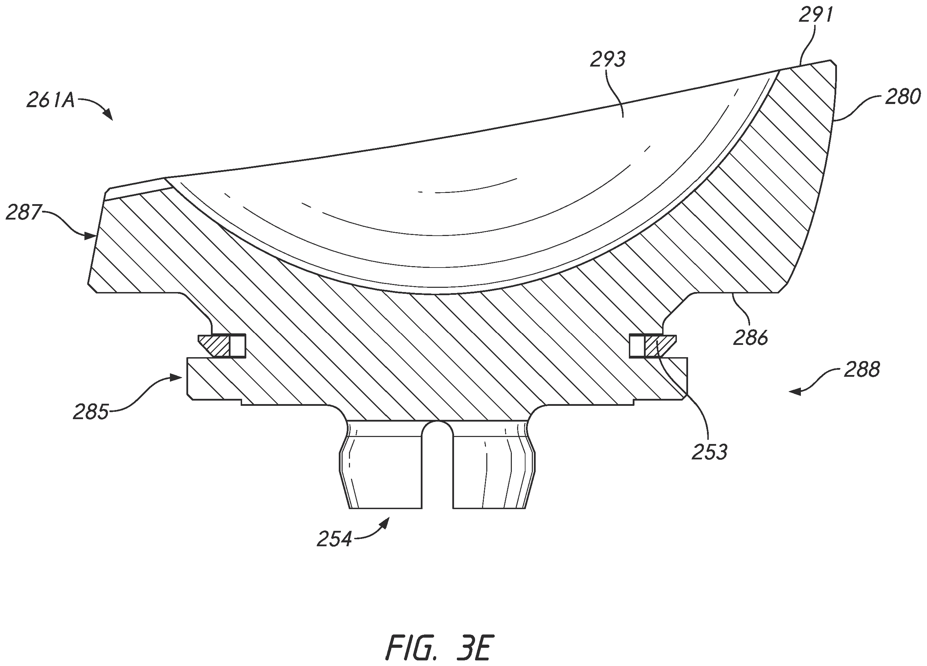

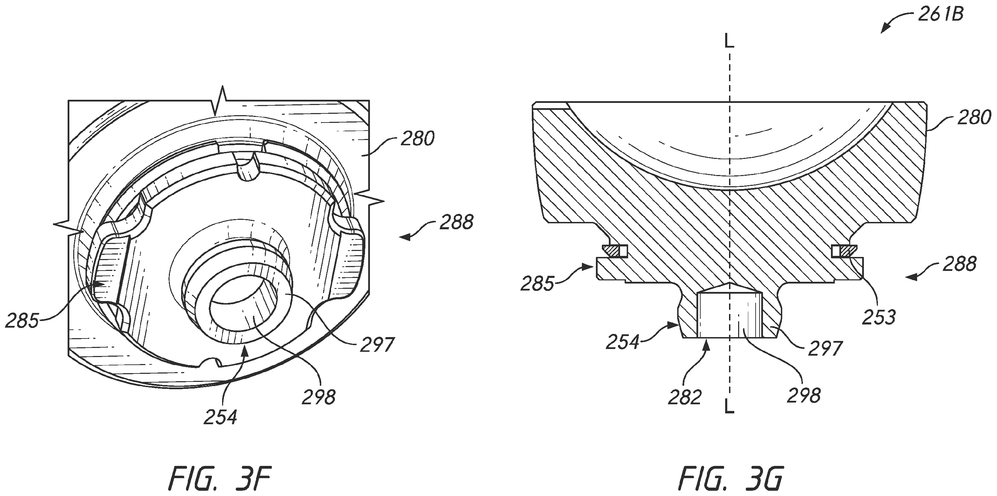

FIGS. 3A-3C illustrate an example articular portion 261, sometimes referred to herein as an articular component. The articular portion 261 includes an articular body 280, such as a reverse component having a concave articular surface, but in some procedures, as explained above, the clinician may select an anatomic articular component, such as the anatomic articular component 160 of FIG. 2. In some embodiments, the articular portion 261 may be an articular assembly, e.g., a polymeric articular body 280 and a locking member 253, as described in further detail below. In other embodiments, the articular portion 261 may be a single-piece, e.g. a polymeric articular body 280.

As shown in FIG. 3A, the articular body 280 may include a first or proximal end 281 and a second or distal end 282. The articular body 280 may have an articular surface 293 disposed at the first end 281 and a humeral anchor interface 288 disposed at a second end 282. The humeral anchor interface 288 of the articular body 280 may secure the articular body 280 to the anchor 203.

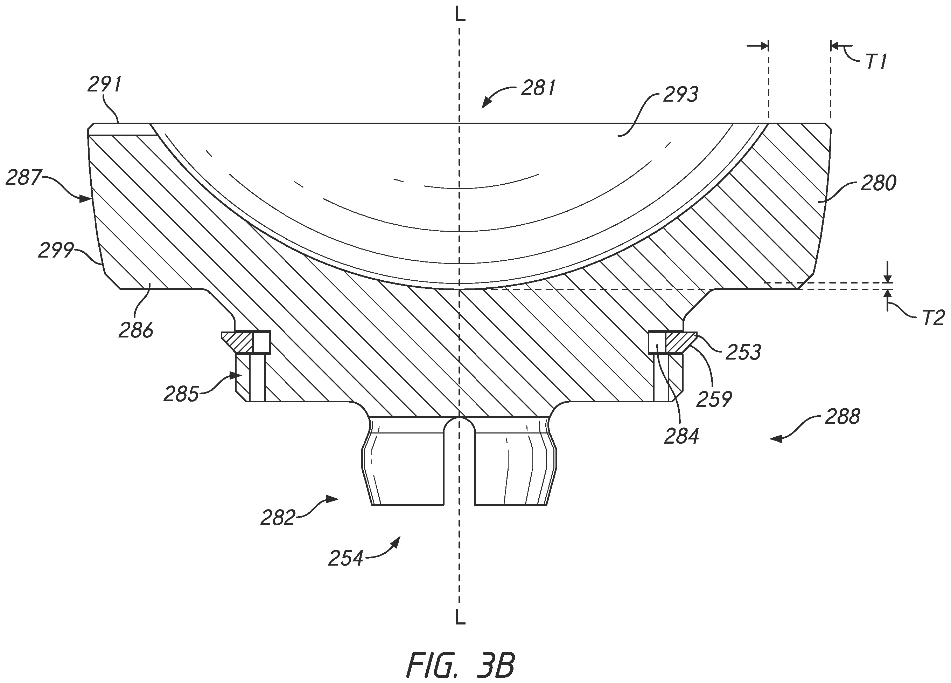

The articular body 280 includes a body portion 287 proximal of the humeral anchor interface 288. The body portion 287 may be defined by an outer wall 299, which may be curved or tapered. A maximum diameter of the body portion 287 may be between 34 mm and 42 mm. The body portion 287 may also include a rim portion 291 between the articular surface 293 and the outer wall 299. The rim portion 291 may have a first thickness T₁ of less than or equal to 5.0 mm or less than or equal to 3.0 mm (see FIG. 3B). The articular body 280 may include a marker 279 to provide an indication of anatomical direction for implantation. For example, the marker 279 may provide an indication of the side of the articular body 280 that should align with the lateral aspect of the humerus. As illustrated in FIG. 3B, the marker 279 may be a divot on the rim portion 291 of the body portion 287. However, the marker 279 may be printed or otherwise visually indicated on the rim portion 291 or elsewhere on the articular body 280.

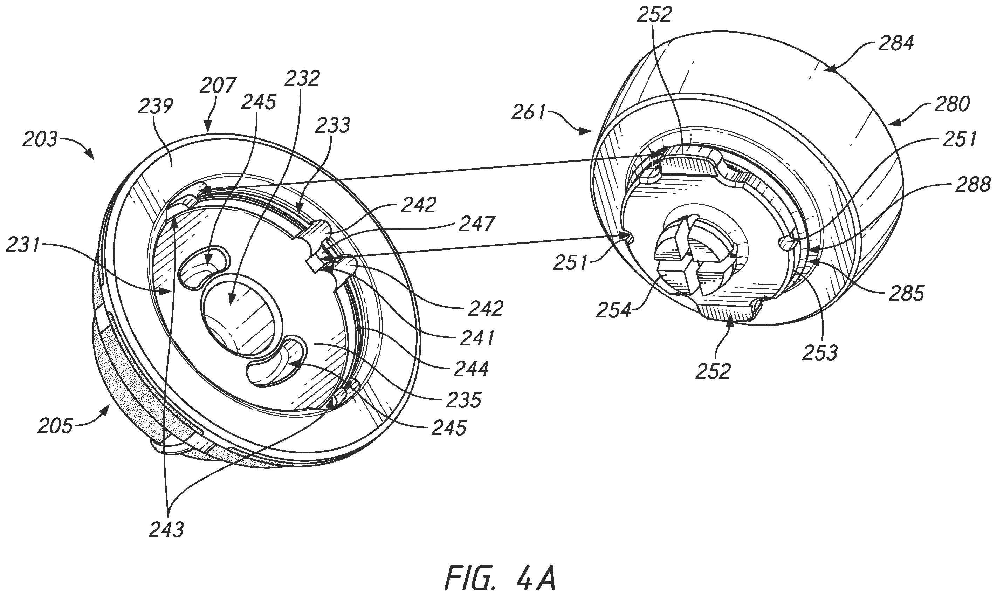

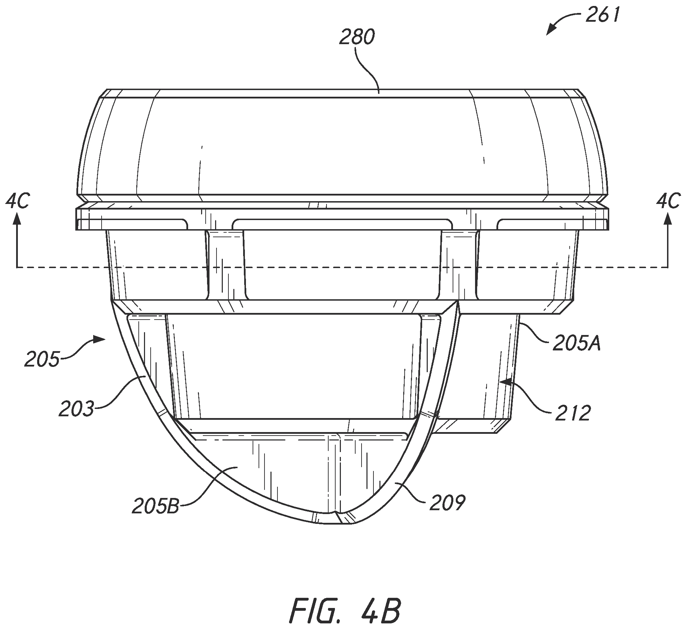

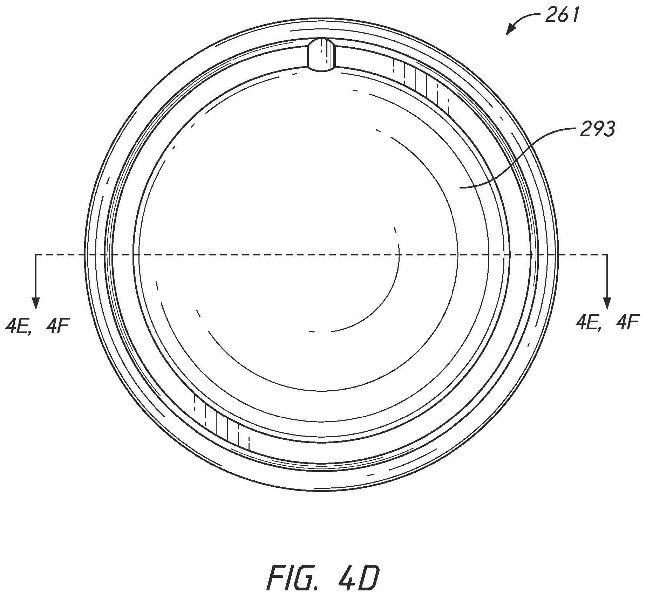

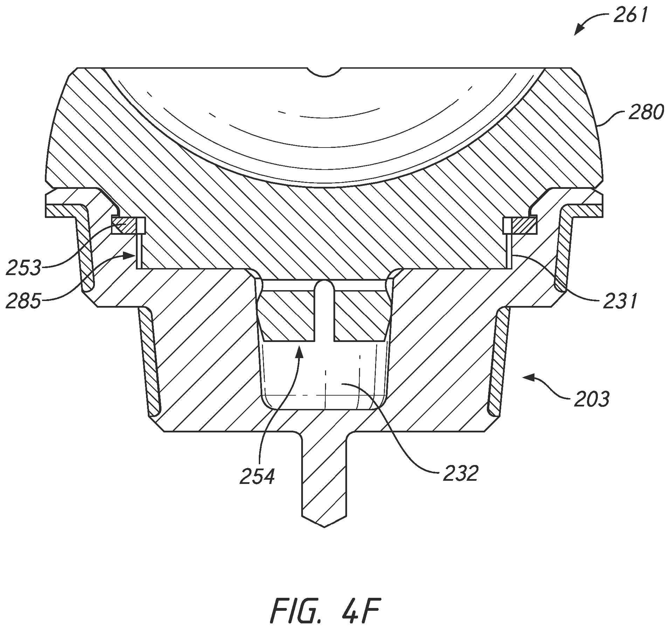

The articular body 280 may also include transverse surface 286 disposed between the first end 281 and the second end 282 of the articular body 280. The transverse surface 286 may define a distal side of the body portion 287. The transverse surface 286 may be configured to overlay a rim or proximal face 239 of an anchor 203 when assembled (see FIGS. 4A & 4F). When assembled with the humeral anchor 203, the body portion 287 may overhang or underhang the anchor 203 by less than or equal to 2.0 mm or less than or equal to about 1.0 mm depending on the size of the humeral assembly.

The body portion 287 may have a second thickness T₂ between a distal most point or apex of the articular surface 293 and the transverse surface 286 (see FIG. 3B). The distal most point or apex of the articular surface 293 is proximal with or in line with the transverse surface 286. In other configurations, the distal most point or apex of the articular surface 293 may be distal to the transverse surface 293. The thickness T₂ may be less than or equal to about 25 mm, less than or equal to about 20 mm, less than or equal to about 15 mm, less than or equal to about 10 mm, less than or equal to about 5 mm, or less than or equal to about 1 mm. In some embodiments, the thickness T₂ may be 0 mm. In use, if the thickness T₂ is not sufficient, the clinician may add thickness using a spacer to achieve the desired thickness between the distal most point of the articular surface 293 and the proximal face 239 of the anchor 203. For example, if the clinician took a low resection or the soft tissue is lax, the clinician may build up the stem using the spacer.

As shown in FIG. 3A-3B, the articular body 280 may be symmetric about the central axis L. For example, the center of the radius of curvature or the center of rotation of the articular surface 293 may be aligned with the central axis L of the articular body 280. Symmetry of the articular body 280 may be desirable for a stemless reverse configuration or a stem having an inclination angle, for example an inclination angle of about 135 degrees. Symmetry of the articular body 280 may be desirable when the scapula form is such that motion of the arm toward the patient does not result in contact between the humeral implant assembly and the scapula bone. Where contact may occur, scapular notching and/or component wear could result. Thus, an angled proximal edge may be used, as discussed further below. An angled insert can be useful for other biomechanical adaptations, as discussed below.

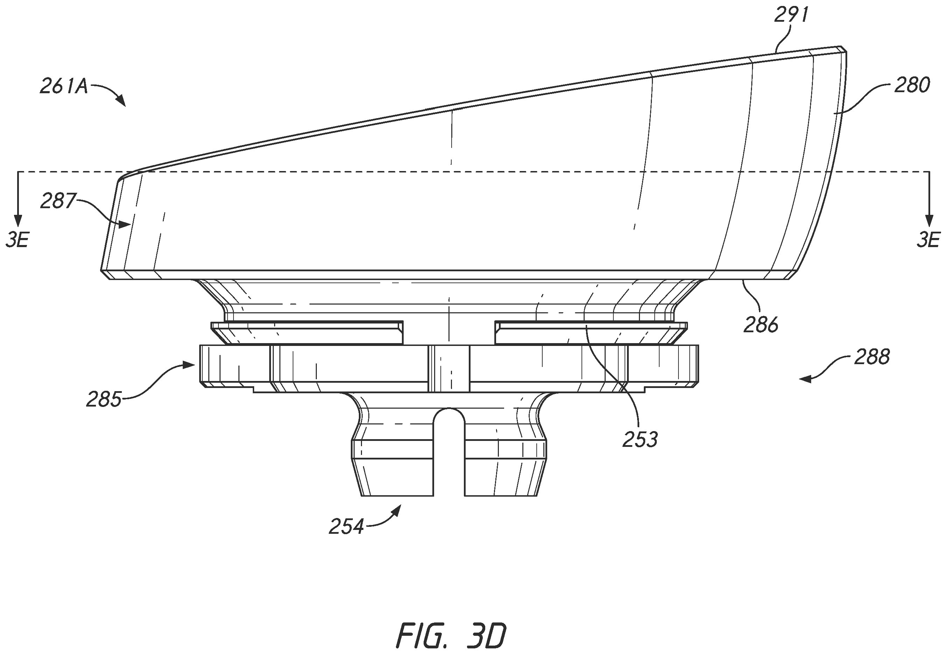

In other configurations, the body portion 287 may be angled to achieve a desired inclination angle. For example, FIGS. 3D-3E illustrate another articular portion 261A in which the proximal portion of the articular body 280 is angled with respect to the transverse surface 286. The center of rotation of the articular surface 293 may be aligned with the central axis L of the articular body 280. In other embodiments, the center of rotation may be angled such that the center of the radius of curvature or the center of rotation is not aligned with the central axis L of the articular body 280. An angle between the transverse surface 286 and the proximal edge or rim portion 291 may be between 7.5 degrees and 17.5 degrees, for example 10 degrees. The angled articular body 280 may be desirable for a stemless anatomic to reverse conversion or a stem having an inclination angle, for example an inclination angle of about 145 degrees.

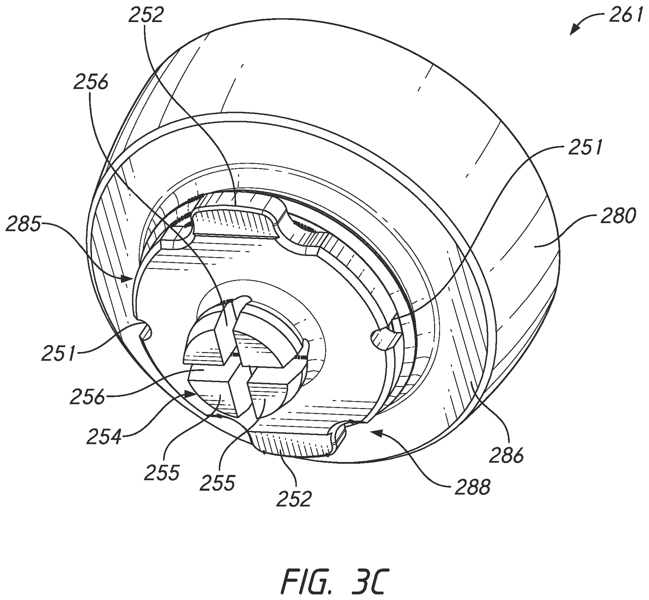

As shown in FIG. 3C, the articular body 280 may include a rotation control zone 285 disposed at a periphery of the articular body 280 between the first end 281 and the second end 282, for example between the transverse surface 286 and the second end 282 of the articular body 280. The rotation control zone 285 may include at least one first alignment feature such as a projection 252. For example, the projection 252 may be a convex tab. The rotational control zone 285 may include a first projection 252 and a second projection 252 circumferentially spaced apart from the first projection 252, for example opposite of the first projection 252. Additionally or alternatively to the first alignment feature, the rotation control zone 285 may include at least one second alignment feature different from the first alignment feature, such as a recess 251. For example, the recess 251 may be a concave slot. The rotational control zone 285 may include a first recess 251 and a second recess 251 circumferentially spaced apart from the first recess 251, for example opposite of the first recess 251. As illustrated, the rotational control zone 285 includes different types of alignment features, such as projections 252 and recesses 251. For example, a projection 252 may extend in a first direction and a recess 251 may extend in a second direction. The first direction may be transverse to the second direction.

As shown in FIG. 3A-3B, the humeral anchor interface 288 may include a channel 284 formed in a surface, for example a circumferential surface, of the humeral anchor interface 288. The channel 284 may be disposed between the first end 281 and the second end 282 of the articular body 280, for example between the rotational control zone 285 and the first end 281 of the articular body 280.

As explained above, the articular portion 261 may be an articular assembly with an articular body 280 and a locking member 253 seated in the channel 284 of the humeral anchor interface 288. The locking member 253 may reversibly secure the articular body 280 against the anchor 203. As shown in FIG. 3A, the locking member 253 may be a locking ring with a discontinuity 258 (e.g., a C-ring) to facilitate radial compression of the locking member 253. The locking member 253 may include a chamfered proximal and/or distal edge to facilitate insertion of the articular portion 261 into the anchor 203. For example, as shown in FIG. 3B, the locking member 253 has a chamfered distal edge 259.

The humeral anchor interface 288 may include a counter-load or deflectable portion 254 projecting distally of the rotation control zone 285 and/or at the second end 282 of the articular body 280. At least a distal portion of the deflectable portion 254 may include a frusto-conical or tapered surface 257 to facilitate insertion into the anchor 203.

The deflectable portion 254 may include at least two sections 255, for example three sections or four sections, cantilevered from a central portion of the articular body 280 to the second end 282 of the articular body 280. The deflectable portion 254 may also include a compression slot 256 disposed between each of the at least two sections 255. For example, as shown in FIG. 3B, the deflectable portion 254 may include four sections 255 separated by intersecting compression slots 256. The compression slots 256 enable the deflectable portion 254 to be compressible in a direction transverse to the longitudinal axis L or compressible in an annular direction.

Other humeral anchor interfaces 288 may also be provided to the articular body 280. For example, FIGS. 3F-3G illustrate a humeral portion 261B in which the humeral anchor interface 288 includes a deflectable portion 254. The deflectable portion 254 may be a compressible plug that exhibits annular compression. The deflectable portion 254 may include a projection 297 having a blind hole 298 extending proximally from the second end 282 and through the projection 297. The blind hole 298 may extend along a central axis L of the articular body 280. The projection 297 may define a continuous periphery without any gaps, slots, or other discontinuities. At least a distal portion of the deflectable portion 254 may include a frusto-conical or tapered surface to facilitate insertion into the humeral anchor 203.

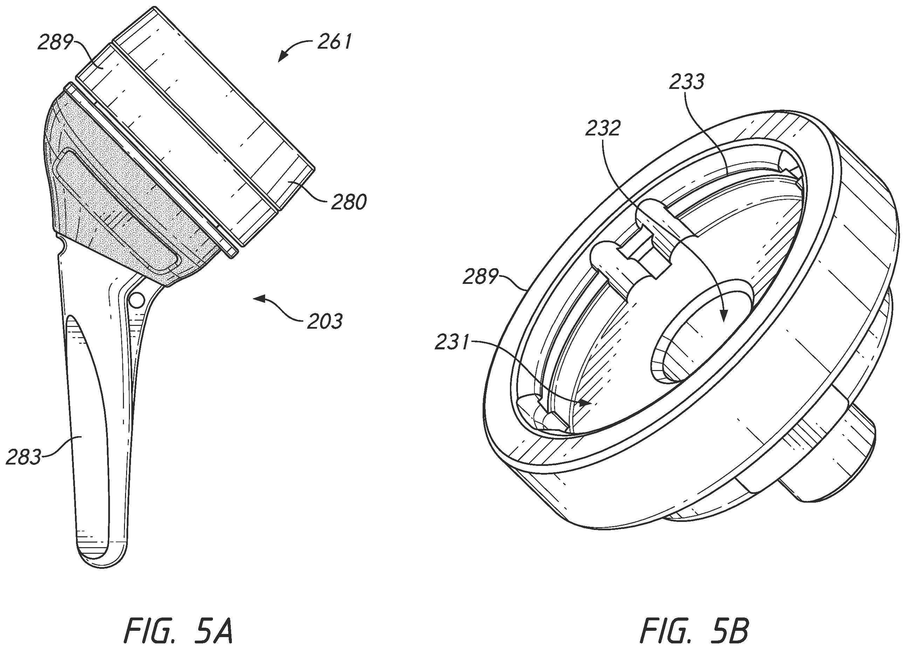

FIGS. 4A-4F illustrate an example of a humeral assembly including an anchor 203 and the articular portion 261. As illustrated, the anchor 203 is stemless, but the humeral anchor could also include a stem (see FIG. 5A). Any of the features described herein with respect to the stemless anchor 203 may be applied to a humeral anchor including a stem or a tray. In some embodiments, the anchor 203 may be monolithic or a single piece. In other embodiments, the anchor 203 may include a first or anchor portion and a second or tray portion adapted for connection to the stemless or stemmed anchor (see FIG. 5B). A single articular portion 261 may be compatible with each of the stemless anchor, stemmed anchor, and/or tray.

As shown in FIG. 4A, a first recess 231 can extend distally from a proximal face 239 of the anchor 203 and into the proximal portion 207. The first recess 231 can be sized and shaped to receive a distal or lateral portion of the articular body 280. As shown in FIG. 4A, the first recess 231 can be disposed in the proximal portion 207 of the anchor 203. A second recess 232 can extend distally from the first recess 231 into a first section 205A of the anchor 203. The first and second recesses 231, 232 can have different volumes. For example, the volume of the first recess 231 (and/or a diameter or major lateral dimension of the first recess 231) can be larger than the volume of the second recess 232 (and/or a diameter or major lateral dimension of the second recess 232). Thus, the combined space formed by the recesses 231, 232 can be larger toward the proximal face 239 of the anchor 203 and smaller toward a distal end 205 of the anchor 203.

As shown in FIG. 4A, the anchor 203 can include an inner periphery 233 disposed about the first recess 231 adjacent to the proximal face 239 of the anchor 203. The inner periphery 233 can be a surface portion extending from the distal interior surface 235 to the proximal face 239 of the anchor 203. The inner periphery 233 can include one or more alignment features configured to interface with corresponding features of the rotational control zone 285 of the articular portion 261. For example, the anchor 203 may include one or a plurality of concave locking features 243 disposed in the inner periphery 233 and/or one or a plurality of convex locking features 241 disposed in the inner periphery 233.

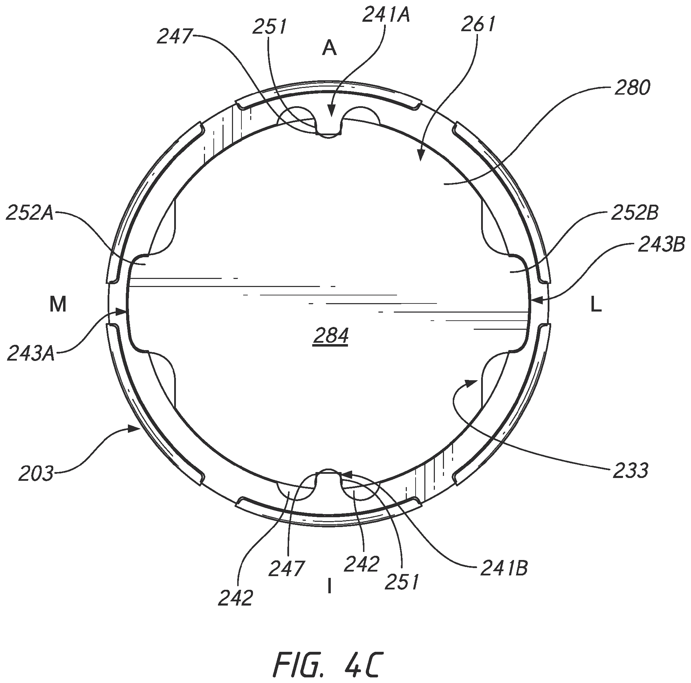

As illustrated in FIG. 4A, the anchor 203 may include a plurality, e.g., two or a pair, of the concave locking features 243 spaced apart from one another along the inner periphery 233. The concave locking features 243 can be circumferentially separated, for example disposed opposite one another across the recess 231 on the inner periphery 233. As shown in FIG. 4C, a first concave locking feature 243A can be disposed at a medial portion M of the anchor 203 and a second concave locking feature 243B can be disposed at a lateral portion L of the anchor 203. In other examples, the first concave locking feature 243A can be disposed at an anterior portion of the anchor 203 and the second locking feature 243B can be disposed at a posterior portion of the anchor 203. An angle can be defined between the concave locking features 243A, 243B, e.g., 180 degrees, 120 degrees, 90 degrees, 60 degrees or other angular separation therebetween. More than two concave locking features 243A or 243B can be provided, e.g., three at 120 degree spacing, four at 90 degree spacing, six at 60 degree spacing. The spacing between the locking features 243A, 243B can be unequal in some embodiments.

Additionally or alternatively to the concave locking features 243, the inner periphery 233 may include plurality, e.g., two or a pair, of convex locking features 241 circumferentially separated from one another, for example disposed opposite one another. As shown in FIG. 4C, a first convex locking feature 241A can be disposed at an anterior portion A of the anchor 203, and a second convex locking feature 241B be disposed at a posterior portion P of the anchor 203. Where both the concave locking features 243 and the convex locking features 241 are present, the concave locking feature 243 can be circumferentially spaced apart from the convex locking feature 241.

As shown in FIGS. 4A and 4C, the convex locking features 241 can include a projection 247 extending radially inward relative to the inner periphery 233 towards the first recess 231. The projection 247 can be elongate with a longitudinal direction oriented proximal-distal in the first recess 231, e.g., parallel to a direction of insertion of the articular portion 261. The projection 247 can extend toward a central portion of the first recess 231 from an adjacent portion of the periphery 233. Portions of the periphery 233 adjacent to the projection 247 can be concave in structure facing toward the first recess 231 relative to the projection 247. For example, each convex locking feature 241 can be adjacent to a pair of concave recesses 242 formed in the inner periphery 233. As with the concave locking features 243, the convex locking features 241 can be sized relative to corresponding locking features of the articular portion 261 that provide an interference connection between the articular portion 261 and the anchor 203.

As shown in in FIG. 4A, the inner periphery 233 may include a channel 244 extending circumferentially along the inner periphery 233. The channel 244 may be distal of the proximal face 239. The clearance between the proximal face 239 and the channel 244 may be less than or equal to 3.0 mm. The channel 244 can include a plurality of segments disposed circumferentially between the concave locking features 243 and the convex locking features 241 (see FIG. 4A). The channel 244 can include any suitable number of segments, for example, four, six, etc. As explained below, the channel 244 can be sized relative to the locking member 253 of the articular portion 261 to provide a snap or interference fit with the locking member 253. In various embodiments, the channel 244 can include a distally-facing surface that can secure the locking member 253 of the articular portion 261 to the anchor 203.

In use, when the clinician inserts the articular portion 261 into the anchor 203, the clinician can align the humeral anchor interface 288 of the articular body 280 relative to the first recess 231 of the anchor 203, for example using the rotational control zone 285. The articular portion 261 may include a first rotational alignment feature (e.g., a projection 252 or a concave slot 251) with a second rotational alignment feature (e.g., the negative of the first rotational alignment feature) disposed in the recess of the anchor 203. When assembled, the engagement between the rotational control zone and the corresponding features of anchor 203 can also serve as anti-rotation features to inhibit relative rotation between the anchor 203 and the articular portion 261.

When properly aligned, at least one projection 252A, 252B of the humeral anchor interface 288 interfaces with the corresponding concave locking feature 243A, 243B of the anchor 203 and/or at least one concave slot 251 of the humeral anchor interface 288 interfaces with the corresponding convex locking features 241A, 241B of the anchor 203 (see FIG. 4C). The rotational control zone 285 of the articular portion 261 and the inner periphery 233 of the anchor 203 can be dimensioned such that, upon insertion of the articular portion 261 into the first recess 231, an interference or friction fit is formed between the articular portion 261 and the anchor 203. For example, the concave locking features 243 can be sized relative to corresponding projections 252 of the articular portion 261 to provide an interference connection between the articular portion 261 and the anchor 203. Such interference fit can include an aspect of the concave locking features 243 being smaller than a corresponding exterior surface of the articular portion 261. As another example, the interference fit can include an aspect of the convex locking features 241 being smaller than a corresponding exterior surface of the articular component 261, for example, the projection 247 can extend into and engage a corresponding exterior surface of the articular component 261.

In embodiments where one or a plurality of alignment or locking features have different shapes and/or sizes, the rotational position can be more easily confirmed intraoperatively. For example, the projection 252 can be visually confirmed to be rotationally positioned correctly relative to the corresponding concave locking features 243 and/or the concave slot 251 can be visually confirmed to be rotationally positioned correctly relative to the corresponding convex locking feature 241. By providing two opposite projections 252, only two rotational positions can result in securing the articular portion 261 to the anchor 203. In some cases, these two positions provide identical biomechanics of the shoulder joint when assembled. The two positions are rotationally symmetric. In other embodiments the two positions provide two options for biomechanics such that the surgeon can select among two positions of the articular component 280 relative to the anchor 203. In a first rotation position, a first projection 252A is positioned in a superiorly positioned first concave recess 243A and a second projection 252B is positioned in an inferiorly positioned second concave recess 243B. In a second rotational position, the first projection 252A is positioned in the inferiorly positioned second concave recess 243B and the second projection 252B is positioned in the superiorly positioned first concave recess 243B. Different numbers of alignment or locking features are also envisioned. For example, there may only be a single alignment feature on the rotational control zone 285 to provide the correct rotational position.

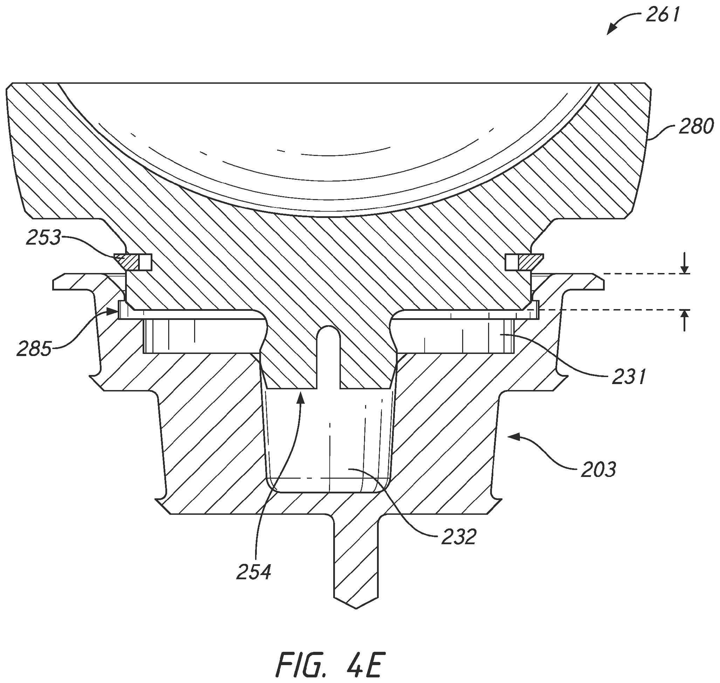

Upon advancement of the articular portion 261 into the anchor 203, a counter-load projection of the articular portion 261 is disposed within the recess of the anchor 203 (see FIG. 4E). The first contact between the articular portion 261 and the anchor 203 may be between a distal portion of the deflectable portion 254 and a proximal portion of the second recess 232. When the articular portion 261 is coupled to the anchor 203, the deflectable portion 254 may be positioned in the second recess 232 of the anchor 203. The articular portion 261 may be advanced until deflectable portion 254 interfaces with a surface surrounding the second recess 232. The deflectable portion 254 may interface with the surface surrounding the second recess 232 before the locking member 253 engages the channel 284 of the anchor 203. For example, the articular portion 261 may be advanced until tapered outer surfaces 257 of the deflectable portion 254 interface with a tapered surface of the second recess 232. Upon contact, sections of the deflectable portion 254 move toward each other across compression slots 256 thereof. When the deflectable portion 254 of the articular body 280 contacts the surface defining the second recess 232, the deflectable portion 254 may be circumferentially deflected by a load applied by or from the surface defining the second recess 232. This deflection by the deflectable portion 254 reduces, minimizes, or eliminates motion, even micro-motion, of the articular portion 261 relative to the anchor 203. When the humeral assembly is assembled, the deflectable portion 254 is sufficiently deflected to cause a load to be applied in a direction opposite to the direction of advancement. The deflectable portion 254 may also provide a load between the locking member 253 and the peripheral portion 233 of the first recess 231.

The articular assembly may be further advanced until a locking member 253 of the articular portion 261 is deflected within a channel 244 formed in the anchor 203 (see FIG. 4F). The locking member 253 can serve to lock the articular portion 261 into the anchor 203 and to prevent the articular portion 261 from translating vertically outward from the anchor 203. The locking member 253 may transition between an at-rest configuration prior to insertion of the articular portion 261 into the anchor 203 and a compressed configuration when the humeral assembly is assembled. In the compressed configuration, the locking member 253 may be radially compressed compared to the at-rest configuration. In the at-rest configuration, an inner periphery of the locking member may be disposed within the channel 284 and the outer periphery of the locking member 253 may be disposed outside the channel 284 (see FIG. 4A). In the compressed configuration, the inner periphery of the locking member 253 may be disposed within the channel 284 and an outer periphery of the locking member 253 may be disposed in a channel 244 of the humeral anchor 203 (see FIG. 4F).

Although the above examples are described with respect to a stemless anchor, the features of the anchor 203 may be applied to an anchor having a stem configured to extend into a humeral diaphysis, a fracture stem or a modular component such as a tray or other spacer. For example, FIG. 5A shows a humeral assembly having a stem. Similar to the stemless humeral assemblies, the stemmed humeral assembly of FIG. 5A may include an anchor 203 and an articular portion 261. As shown in FIG. 5A, the anchor 203 may include a tray 289 mounted to a metaphyseal portion of a stem 283, for example by matching tapers forming a Morse taper connection. The articular portion 261, which may include any of the features described above, may be mounted to the tray 289, for example using any of the humeral anchor interface features described above.

As shown in FIG. 5B, the tray 289 includes similar internal features as the stemless anchor 203 shown in FIG. 4A. For example, the tray 289 includes a first recess 231 and a second recess 232 extending distally from the first recess 231. The tray 289 also includes an inner periphery 233 disposed about the first recess 231. The inner periphery 233 may include one or more alignment or locking features configured to interface with the rotational control zone 285 of the articular portion 261, as described above. The inner periphery 233 may include a circumferential channel 244 extending circumferentially along the inner periphery 233. The channel 244 may be sized relative to the locking member 253 of the articular portion 261 to provide a snap or interference fit with the locking member 253.

As discussed above, the clinician may optionally provide a tray 289 or other spacer to fill the soft tissue space. For example, if the clinician took a low resection or the soft tissue is lax, the clinician may build up the stem using the tray 289 or other spacer. The tray 289 or the spacer will build up the thickness between the distal most point of the articular surface and the proximal face of the stem 283.

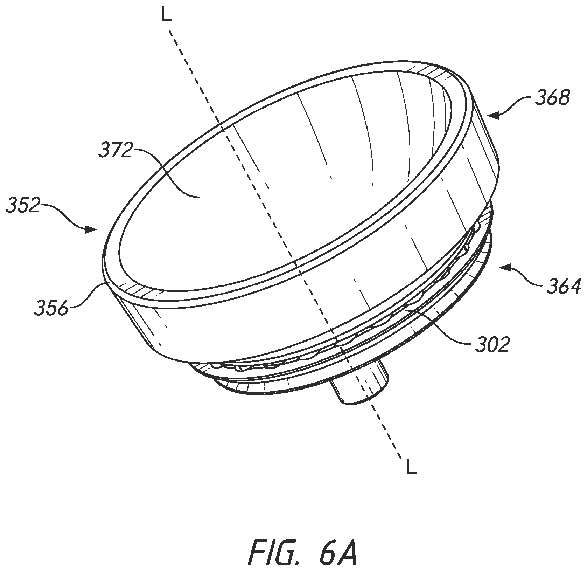

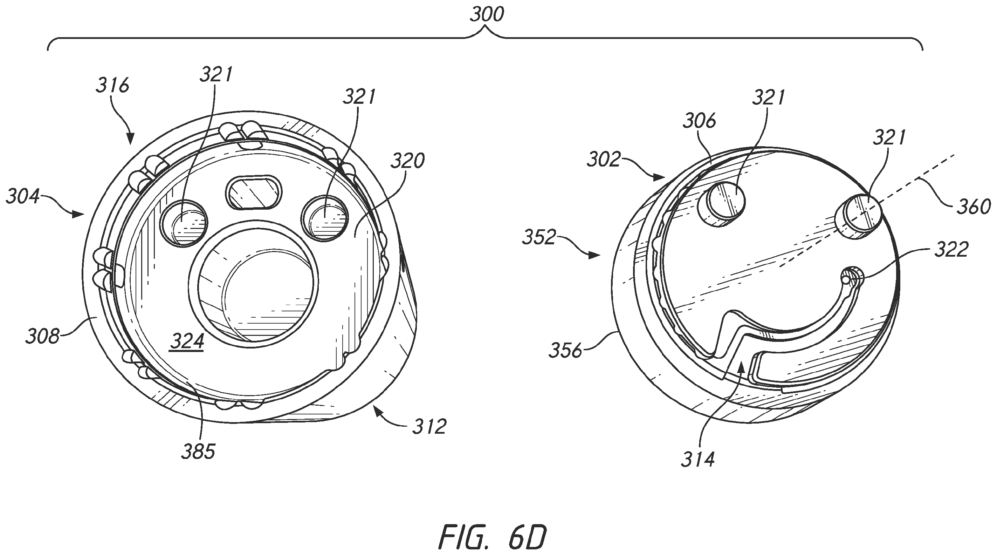

FIGS. 6A-6G illustrate another articular portion 352 configured to be coupled to an anchor 304. The articular potion 352 can be configured as an articular component or assembly including functionally and physically distinct components. The anchor 304 may include any of the features described above with respect to any of the anchors 103, 203, 113 or 140. For example, the anchor 203 may be a stemless anchor. A single articular portion 352 may be compatible with each of a stemless anchor, a stemmed anchor, and/or a tray. The anchor 304 has a body portion 308, for example a metallic body (see FIG. 6D). The body portion 308 has a bone engaging side 312 configured to be placed against the bone and an assembly side 316 opposite the bone engaging side. The bone engaging side 312 can form a part of any of the anchors described herein, including a stemless anchor or a stemmed anchor. The assembly side 316 has a mounting area 324, which can include one or more recesses extending toward the bone engaging side, configured to receive at least a portion of the articular portion 352. The mounting area 324 may include a channel 385 disposed about a periphery thereof.

FIGS. 6A-6B provide different views of the articular portion 352. The articular portion 352 may include any of the features of the articular portion 261. As described above, the articular portion 352 includes an articular body 356, such as a reverse component having a concave articular surface, but in some procedures, as explained above, the clinician may select an anatomic articular component, such as the anatomic articular component 160 of FIG. 2. In some embodiments, the articular portion 352 may be an articular assembly, e.g., a polymeric articular body 356 and a locking member 302, as described in further detail below. In other embodiments, the articular portion 352 may be a single-piece, e.g. a polymeric articular body 356.

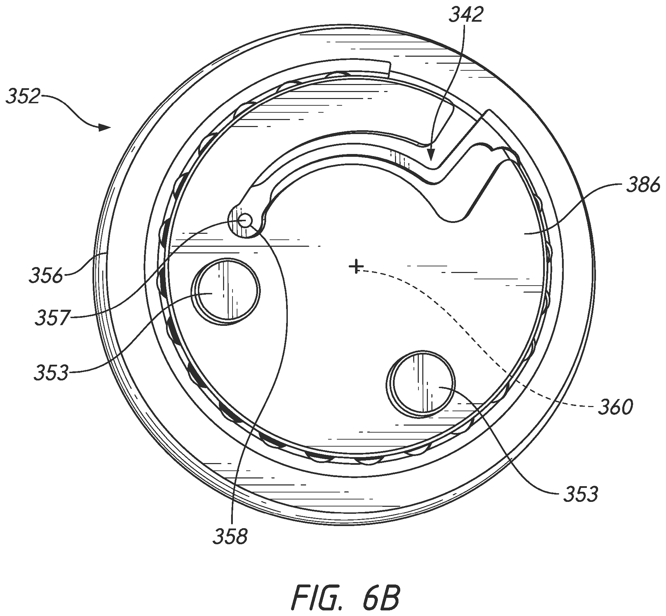

As shown in FIG. 6A, the articular body 356 includes a first or distal portion 364 and a second or proximal portion 368. The articular body 356 has an articular surface 372 disposed in the second portion 368. The first portion 364 of the articular body 356 includes a distal surface 386 configured to be disposed in the mounting area 324 of the anchor 304. The first portion 364 of the articular body 356 may include one or more pegs 353 from the distal surface 386 (see FIG. 6B). The one or more pegs 353 may correspond to one or more corresponding recesses 321 in a proximal face 320 of the bone anchor 304 (see FIG. 6D). At least one peg 353 may be positioned off-center, displaced from the center 360 of the articular body 356. In one case, multiple pegs 353 are provided and each is off-center. Some variation may include a central recess disposed in the proximal face 320 of the bone anchor 304, e.g., for mounting an anatomic articular component, such as the component 160. The pegs 353 facilitate rotational alignment between the articular portion 352 and the anchor 304. In some embodiments, the articular body 356 may include a central peg or other projection extending from the distal surface 386 of the articular body 356. Any of the pegs 353 may engage the anchor 304, for example by matching tapers forming a Morse taper connection. Although the illustrated articular body 356 includes pegs 353, in other embodiments, the articular body 356 may include one or more recesses extending proximally from the distal surface 368. The recesses may corresponding to one or more pegs extending proximally from the proximal face 320 of the anchor 304. In another variation, each of the anchor 304 and the articular body 356 has at least one peg and at least one recess to provide for an advantageous combination of connection features.

As shown in FIG. 6A, the second portion 368 of the articular body 356 may include a channel 384 disposed between the articular surface 372 and the distal surface 386. The channel 384 may be formed in a surface, for example a side peripheral or circumferential surface, of the second portion 368. As shown in FIG. 6B, the second portion 368 may include a recess 342 on a distal surface 386 of the articular body 356. The recess 342 may extend from the channel 384. For example, the recess 342 may extend proximally from the distal surface 386 of the articular body 356 to a depth of the channel 384.

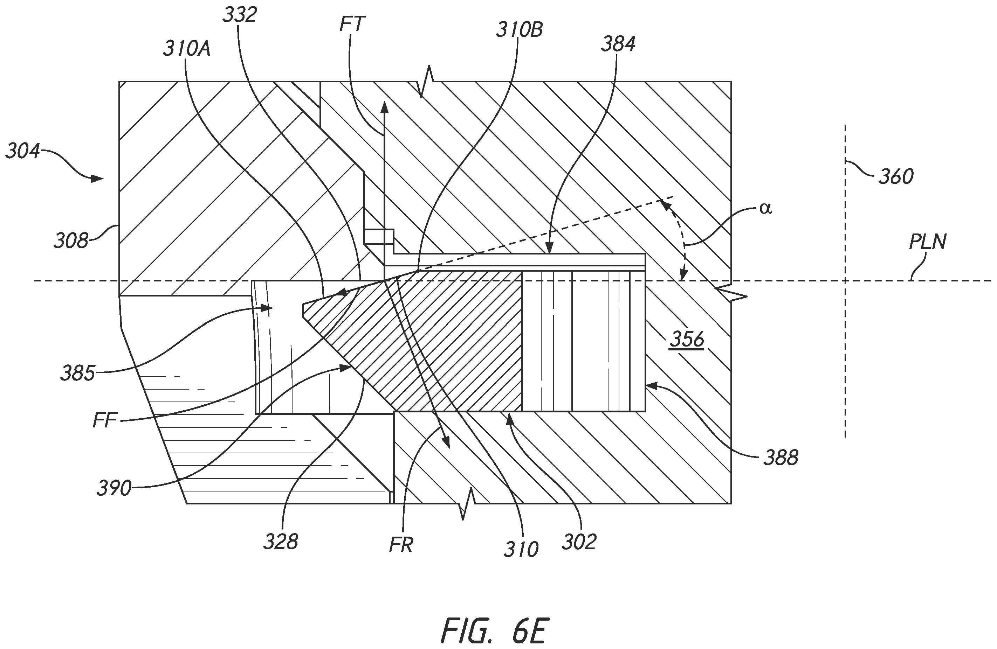

As explained above, the articular portion 352 may include a locking member 302 to ensure mechanical fixation between the articular body 356 and the anchor 304. The locking member 302 may be constructed of a resilient material such as titanium or another resilient metal. As shown in FIGS. 6A-6B, the locking member 302 may be disposed about the articular body 356, for example within the channel 384 of the second portion 368. The locking member 302 may reversibly secure the articular body 356 against the anchor 304 with a simple impaction load by an impactor and mallet but without the use of any other tools for assembling the articular body 356 to the anchor 304. When the articular portion 352 is coupled to the anchor 304, the locking member 302 is deflected into the anchor channel 385 such that an outer periphery 390 of the locking member 302 is disposed within the anchor channel 385 and an inner periphery 388 is disposed within the articular body channel 384 (see FIG. 6E).

The locking member 302 may include an arcuate member 306 disposed at least partially about the first portion 364 of the articular body 356, for example at least partially within the articular body channel 384. As shown in FIGS. 6B-6C, the arcuate member 306 may include a discontinuity 374 between a first end 366 of the arcuate member 306 and a second end 378 of the arcuate member to facilitate radial compression of the locking member 302.

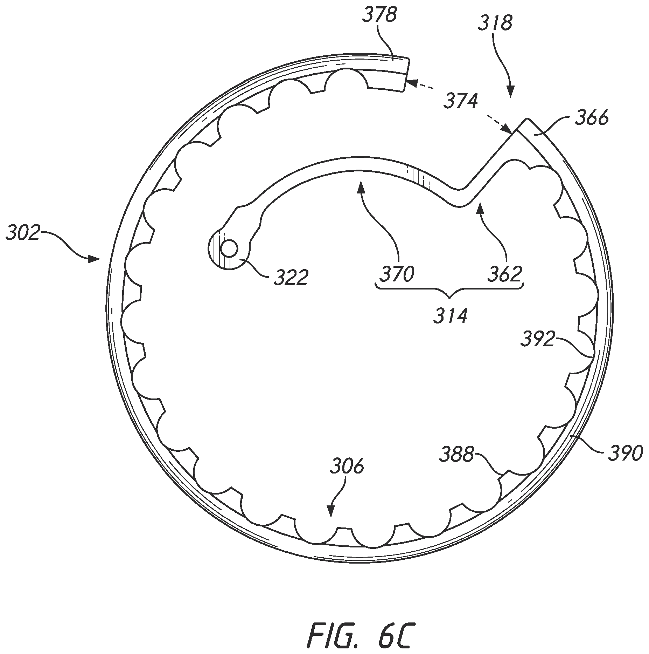

The inner periphery 388 and/or the outer periphery 390 of the locking member 302 may include one or more stress reduction features 392. For example, as shown in FIG. 6C, the arcuate member 306 includes a scalloped edge or a series of cut outs along at least a portion or the entire inner periphery 388, but in other example, the scalloped edge may be along the outer periphery 390. Although the figures illustrate the scalloped edge, other stress reduction features may include a reduction in thickness, measured between the proximal and distal surfaces of the locking member 302. The stress reduction features 392 facilitate bending and deflection of the arcuate member 306 to avoid plastic deformation during assembly. The stress reduction features 392 also help maintain the integrity of the locking member 302 when impaction forces are applied to the humeral assembly.

As shown in FIG. 6E, the outer periphery 390 of the locking member 302 may have a first or distal edge 328 and a second or proximal edge 310 disposed at an angle relative to the first edge 328 to form an apex. The second or proximal edge 310 may be disposed at an angle α relative to a plane PLN disposed perpendicular to the central insertion axis 360 or along the anchor retention surface 322. The anchor retention surface 322 defines an upper edge of the anchor channel 385. The angle α may be greater than 0 degrees and less than or equal to 20 degrees, for example about 15 degrees. For a locking member material with a higher friction factor, the angle α could be greater than 20 degrees. For example, the angle α could be less than or equal to about 45 degrees or less than or equal to about 30 degrees.

As shown in FIG. 6E, the second edge 310 of the locking member 302 may include a first portion 310A and a second portion 310B. To maintain fixation between the articular body 356 and the anchor 304, at least part of the first portion 310A of the second edge 310 has to extend into the channel 385 in the anchor 304. As the articular portion 352 is inserted into the anchor 304, the distal edge 328 of the locking member 302 is advanced toward the distal edge of the channel 385. After impaction, the locking member 302 elastically recovers such that the proximal edge 310 contacts the retention surface 322. When the articular body 356 is coupled with the anchor 304, the first portion 310A is disposed farther from the articular surface 372 in the direction of the central insertion axis 360 than the retention surface 322 of the anchor 304. A second portion 310B of the second edge 310 is at least along the same plane as the retention surface 332 of the anchor channel 385 or disposed closer to the articular surface 372 in the direction of the central insertion axis 360 than the retention surface 332.

When the locking member 302 is deflected into the anchor channel 352, there is an interference fit between the anchor 304 and the locking member 302. An angle α, between the second edge 310 of the locking member 302 and plane PLN, that is less than or equal to about 45 degrees (or less than or equal to about 30 degrees or less than or equal to about 20 degrees, for example less than or equal to about 15 degrees), will maintain contact between the locking member 302 and the anchor 304 regardless of a traction force F_{T} pulling apart the articular body 352 and the anchor 304. As shown in FIG. 6E, the friction force F_{F} between the locking member 302 and the anchor 304 will prevent release of the locking member 302 from the anchor channel 385, while the reaction force F_{R} will prevent the locking member 302 from sliding farther into the anchor channel 385. The combination of the friction force F_{F} and the reaction force F_{R} reduces or eliminates motion between the articular body 356 and the anchor 304. For example, the locking member 302 allows no more than 0.05 mm movement, or even no movement, in a longitudinal and/or transverse direction, between the anchor 304 and the articular body 356. The sloped locking member 302 allows the humeral assembly to accommodate the high forces imposed on shoulder joints without risk of disassembly or removing manufacturing clearance.

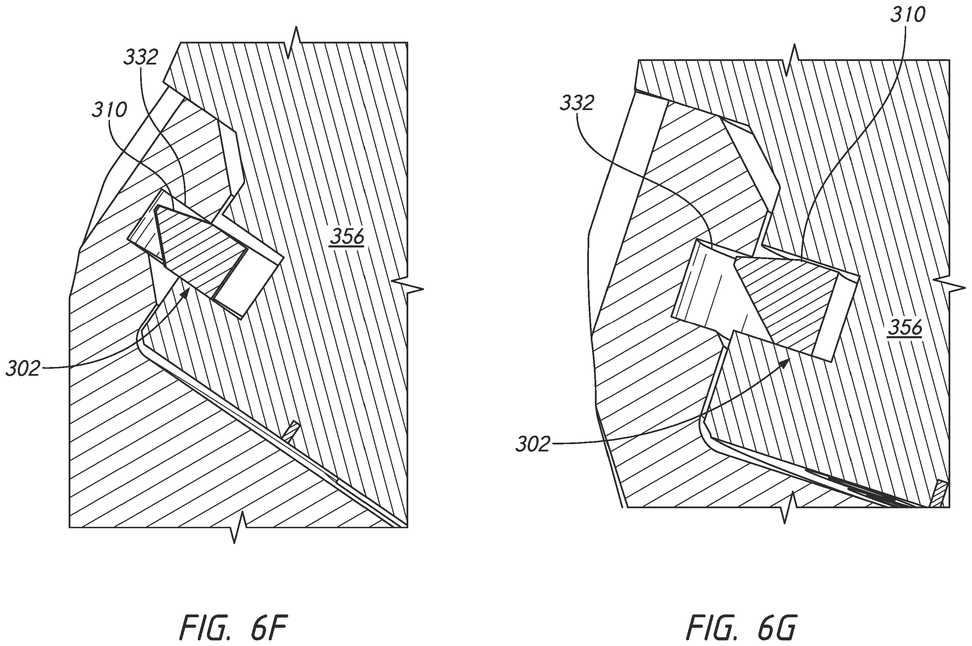

The shape of the outer periphery 388 also enables the locking member 302 to tolerate a range in clearance between the anchor channel 385 and the articular body channel 384. For example, the locking member 302 maintains fixation between the articular body 356 and the bone anchor 304 regardless if there is maximal clearance between the anchor channel 385 and the articular body channel 384 (or other relatively large clearance as in FIG. 6F) or minimal clearance between the anchor channel 385 and the articular body channel 384 (or other relatively small clearance as in FIG. 6G).

The locking member 302 may also include a resilient body 314 extending from the arcuate member 306. The resilient body 314 may have a shape generally corresponding to a shape of the recess 342 in the articular body 356. The recess 342 may be configured to receive the resilient body 314 in a limited number of positions or only one position, such as a centered position. The recess 342 can be shaped to accommodate the resilient body 314 while allowing some motion of the body 314 to that the articular body 356 will not impede loading and unloading of the body 314. This feature facilitates proper orientation of the locking member 302 relative to the articular body 356. Proper orientation facilitates easy removal of the locking member 302 as the locking member is always in the same position.

As shown in FIG. 6C, the resilient body 314 includes a first end 318 and a second end 322. The first end 318 of the resilient body 314 may extend from the first end 366 of the arcuate member 306. The second end 322 of the resilient body 314 is disposed radially inward of the arcuate member 306. In this configuration, the second end 378 of the arcuate member 306 forms one free end of the locking member 302 and the second end 322 of the resilient body 314 forms another free end of the locking member 302. The second end 322 of the resilient body 314 can be overlapping with the arcuate member 306 but disposed at an inward radial position.

As shown in FIG. 6C, the resilient body 314 may include a radial portion 362 extending radially inward from the arcuate member 306. The resilient body 314 may also include an arcuate portion 370 extending from the radial portion 362. The arcuate portion 370 may be concentric with the arcuate member 306. For example, the arcuate portion 370 may be radially inward of the arcuate member 306 and span at least a length of the gap 374 in the arcuate member 306.

The locking member 302 may be coupled to body portion 356 to retain the locking member 302 in a pre-defined position and orientation. For example, the second end 322 of the resilient body 314 may be coupled to the body portion 356. As shown in FIG. 6B, the second end 322 of the resilient body may include a first engagement feature 358 coupled to a second engagement feature 357 of the body portion 356. The first engagement feature 358 may include an opening or a peg, and the engagement feature 357 of the body portion 356 may include a negative of the first engagement feature 358. If the engagement feature 357 is a peg, the engagement feature 358 may be an aperture formed in the second end 322.

The second end 322 of the resilient body 314 may be enlarged to form a locator body. The locator body may be disposed in the recess 342 in a pre-defined orientation and/or position relative to the central insertion axis 360. The pre-defined orientation and/or position may be a single orientation and/or position, such as a centered position within the recess 342. In some embodiments the resilient body 314 can have an arcuate portion and the walls of the recess 342 in the portion thereof in which the body is positioned can have an arcuate form. The side edges of the resilient body 314 can be spaced from, e.g., equally spaced from, opposing walls of the recess 342. In some embodiments, the spacing of the resilient body 314 from the walls of the recess 342 can increase along the length of the body in a direction away from the engagement features 357, 358.

As described above, the locking member 302 may be shaped to accommodate a range of clearances between the anchor channel 385 and the articular body channel 384. However, this may cause the locking member 302 to be not centered relative to a central axis 360 of the shoulder implant. The resilient body 314 may configured to center the arcuate member 306 relative to the central insertion axis 360. For example, the resilient body 314 stores strain energy upon application of a deflection force to deflect the locking member 302 away from a centered position (e.g., about center point 360) and releases the strain energy to return the locking member 302 toward the centered position upon removal of the deflection force.

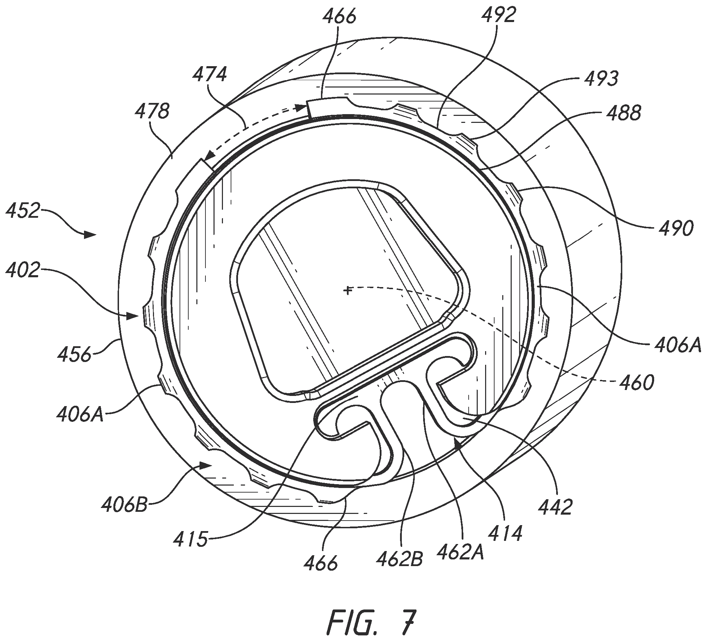

Other configurations of the locking member may achieve one or more of the benefits described above. For example, FIG. 7 illustrates an articular portion 452 including an articular body 456 and a locking member 402. The locking member 402 may include any of the features described above with respect to the locking member 302.

As shown in FIG. 7, the locking member 402 includes an arcuate member 406 disposed at least partially about the articular body 456. The arcuate member 406 may include a discontinuity 474 between a first end 466 of the arcuate member 406 and a second end 478 of the arcuate member to facilitate radial compression of the locking member 402. The inner periphery 488 and/or the outer periphery 490 of the locking member 402 may include one or more stress reduction features 492 to facilitate bending and deflection of the arcuate member 406 to avoid plastic deformation. For example, as illustrated, the arcuate member 406 includes a scalloped edge, a series of cut outs along at least a portion of the outer periphery 488. Although FIG. 7 illustrates the scalloped edge, other stress reduction features are possible such as reduced thickness or in some embodiments a material processing technique is provided along an edge or periphery or between the proximal edge and the distal edge of the locking member 402. The segments 493 between the stress reduction features 492 may include any of the features of the outer periphery 390, described above, to facilitate engagement between the articular portion 452 and the anchor.

As illustrated, the arcuate member 406 includes a first arcuate portion 406A extending from the first end 466 and a second arcuate portion 406B extending from the second end 478. The locking member 402 may also include a resilient body 414 extending between the first arcuate portion 406A and the second arcuate portion 406B. The resilient body 414 includes a base 415 positioned radially inward of the arcuate member 406. The base 415 spans at least a length of the gap 474 in the arcuate member 406 in one embodiment. The resilient body 414 may also include a first radial member 462A extending from the base 402 to the first arcuate portion 406A and a second radial member 462B extending from the base to the second arcuate portion 406B. In one embodiment the base 415 includes an overhang portion 464 that extends away from one of the first radial member 462A, 462B to a wall of the recess 442. In one embodiment the overhang portion 464 spaces the first radial member 462A from the wall of the recess 442 such that the wall does not constrain motion of the member 462A. The base 415 can have an overhang portion 464 at each end to provide this spacing function for both members 462A, 462B.

The resilient body 414 may have a shape generally corresponding to a shape of the recess 442 in the articular body 456. The recess 442 may be configured to receive the resilient body 414 in a limited number of positions or only a single position, such as a centered position. For example, the general form of the recess 442 can be T-shaped. The portion of the recess 442 that receives the base 415 can be wider than a portion of the recess extending from the periphery to the base retaining portion of the recess. Similarly the base 415 of the resilient body 414 can be sider than the combined width of the radial members 462A, 462B. The resilient body 414 can form C-shaped an inverted or reverse or backward C-shaped concavities that can receive a portion of the bottom or distal or medial side of the articular body 456.

The resilient body 414 may configured to center the arcuate member 406 relative to the central insertion axis 460. For example, the resilient body 414 stores strain energy upon application of a deflection force to deflect the locking member 402 away from a centered position (e.g., about center point 460) and releases the strain energy to return the locking member 402 toward the centered position upon removal of the deflection force.

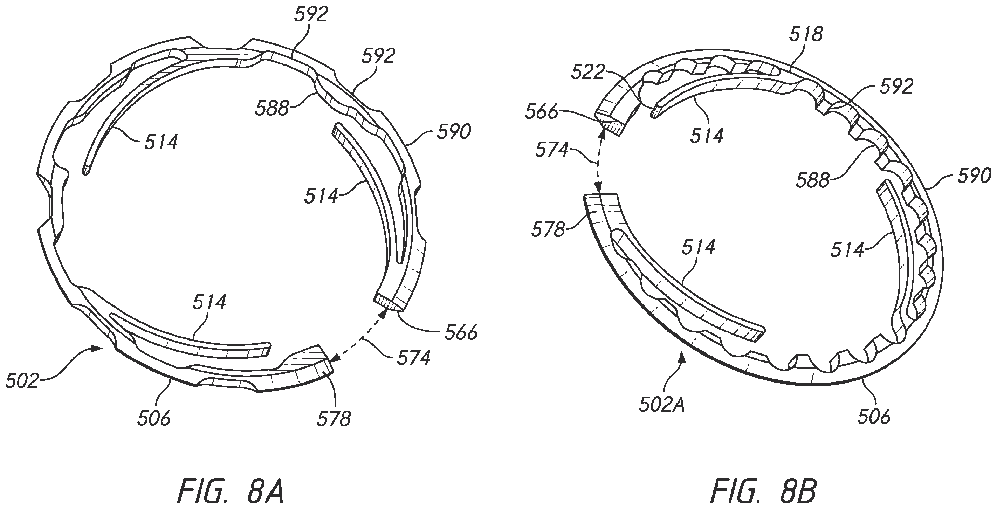

FIG. 8A illustrates another locking member 502 including a different configuration of the resilient body. The locking member 502 may include any of the features described above with respect to the locking members 302, 402.

As illustrated, the locking member 502 includes an arcuate member 506 having a discontinuity 574 between a first end 566 of the arcuate member 506 and a second end 578 of the arcuate member 506 to facilitate radial compression of the locking member 502. The arcuate member 506 may also include one or more stress reduction features 592 disposed along at least a portion of or the entire inner periphery 590 and/or outer periphery 588 of the arcuate member 506. As shown in FIG. 8B, the locking member 502A may only include stress reduction features 592 along only the inner periphery 590 of the arcuate member 506. In another variation the stress reduction features 592 can be located on only the outer periphery 588 of the arcuate member 506. In either configuration, the outer periphery 588 may have a sloped configuration as described above with respect to locking member 302.

The locking member 502 may also include one or more resilient bodies 514 (e.g., at least two resilient bodies or at least three resilient bodies or in one case only three resilient bodies) extending from the inner periphery 590 of the arcuate member 506. Each of the resilient bodies 514 has a first end 518 extending from the arcuate member 506 and a second free end 522 positioned radially inward of the first end 518. The resilient body 514 may have an arcuate shape from the first end 518 to the second end 522. The resilient bodies 514 may be circumferentially spaced apart from each other. For example, the second end 522 of each resilient body 514 may be circumferentially spaced apart from (e.g., equally spaced apart and in the case of only three bodies 514 120 degrees apart from) the first end 518 of the adjacent resilient body 514.

When coupled to the articular body, the resilient bodies 514 may be disposed within an articular body channel. Each resilient body 514 may configured to center the arcuate member 506 relative to the central insertion axis. For example, each resilient body 514 stores strain energy upon application of a deflection force to deflect the locking member 502 away from a centered position and releases the strain energy to return the locking member 502 toward the centered position upon removal of the deflection force. Where more than one resilient body 514 is provided the bodies may work together to cause the locking member 502 to move toward a central position. For example, as stored strain energy of one of the bodies 514 (e.g., the body spanning the 3 o'clock position in FIG. 8A) is released, an adjacent span of the locking member 502 (also at 3 o'clock) may shift away from the body 514 (e.g., the member 502 may move toward the right in FIG. 8A, increasing a gap between the member 502 and the adjacent body 514 at the 3 o'clock position). This may cause a corresponding shifting of another span of the locking member 502 (e.g., at 10 o'clock) toward another of the bodies 514 (e.g., the body 514 connected to the member 502 at 12 o'clock, which extends to a free end located between 9 and 10 o'clock), which can cause strain energy to be stored in the other body 514 (reducing the gap between the locking member 502 and the body 514 at the 10 o'clock position). Storing strain energy in the other resilient body 514 can limit the motion of the locking member 502 (in this example, to the right) such that the resilient body 514 does not over-correct by moving the locking member off-center (e.g., too far to the right).

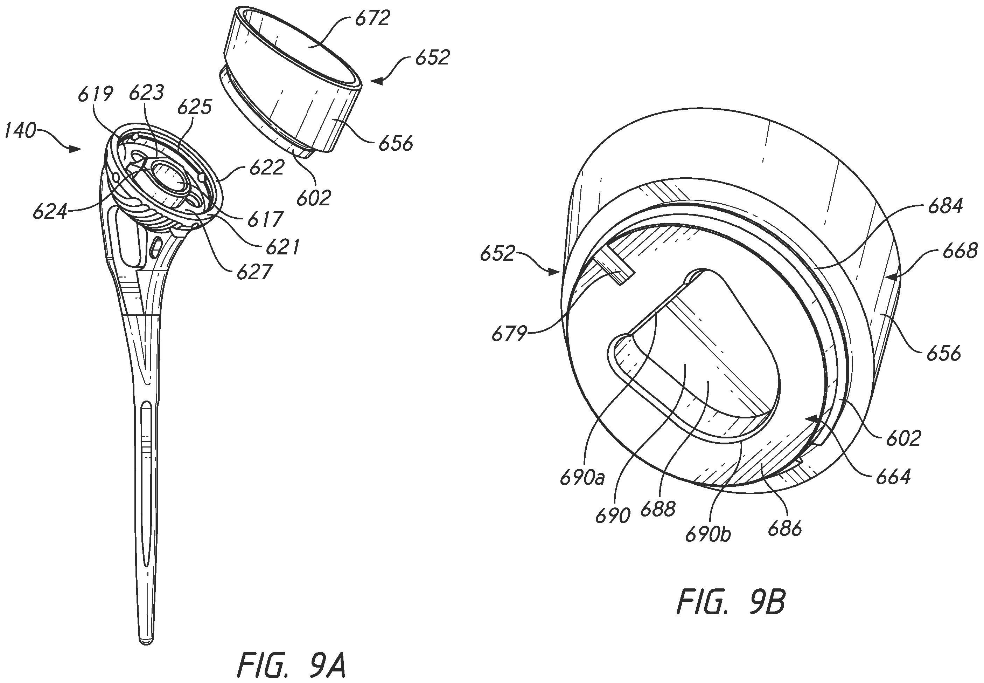

As explained above, for humeral fractures, the kit 100 can also include one or more fracture stems 140. FIG. 9A illustrates another articular portion 652 configured to be coupled to the fracture stem 140. Although FIG. 9A illustrates a fracture stem, the fracture stem may be a stemless anchor or a stemmed anchor including any of the features described above with respect to any of the anchors 103, 203, 113 140, or 304. Thus, the connection features of the fracture stem 140 can be shared among variations of these additional humeral anchors to provide shared components in a variation of the kit 100 where the connection features of the stem 140 are present in other anchors in the kit.

The proximal end of the fracture stem 140 includes a peripheral wall 621 defining a cavity 619. The cavity 619 is radially spaced from and surrounds a hole 617 located at a proximal end of the stem 140. The hole 617 may be at least partially formed in a raised portion 623. The raised position 623 of the hole 617 enables the fracture stem 140 to be compatible with an anatomic articular component that is similar to the anatomic articular component 160 but can exclude the disc or middle portion 162 that is provided in the coupler 168. A modified version of the coupler 160 can provide two adjacent cones without a spacer similar to the disk or middle portion 162. The raised portion 623 extends proximally from the base 627 of the cavity 619. A proximal surface 624 of the raised portion 623 can be in the same plane or substantially the same plane as the proximal surface 622 of the peripheral wall 621. The fracture stem 140 may also include a channel 625 surrounding an inner periphery of the peripheral wall 621.

The articular potion 652 can be configured as an articular component or assembly including functionally and physically distinct components. A single articular portion 652 may be compatible with each of a stemless anchor, a stemmed anchor, and/or a tray. The articular portion 652 may include any of the features described above with respect to the articular portions 261, 352, 452. As described above, the articular portion 652 includes an articular body 656, such as a reverse component having a concave articular surface 672, but in some procedures, as explained above, the clinician may select an anatomic articular component, such as the anatomic articular component 160 of FIG. 2. In some embodiments, the articular portion 652 may be an articular assembly, e.g., a polymeric articular body 656 and a locking member 602, as described in further detail below. In other embodiments, the articular portion 652 may be a single-piece, e.g. a polymeric articular body 656.

As shown in FIG. 9B, the articular body 656 includes a first or distal portion 664 and a second or proximal portion 668. The articular body 656 has an articular surface 672 disposed in the second portion 668. The first portion 664 of the articular body 656 includes a distal surface 686 configured to be disposed in the cavity 619 of the fracture stem 140. For example, the first portion 664 of the articular body 656 may include a recess 690 extending proximally from the distal surface 686. The shape of the recess 690 may correspond to the raised portion 623 in the proximal face of the fracture stem 140 (see FIG. 9A). The recess 690 may be asymmetric about at least one axis to facilitate proper rotational alignment with the raised portion 623, e.g., to allow only one, two or another limited number of rotational positions in which the recess 690 may receive the raised portion 623. For example, as shown in FIG. 9B, the recess 690 may have a straight portion 690a on a lateral side thereof and a curved portion 690b on a medial side thereof. In other embodiments, the recess 690 may be symmetric about all axes, for example circular. When the articular portion 652 is coupled to the fracture stem 140, a surface 688 of the recess 690 overlays the raised portion 623 without filling the hole 617.

The articular body 656 may include a marker 679 to provide an indication of anatomical direction for implantation. For example, the marker 679 may provide an indication of the side of the articular body 656 that should align with the lateral aspect of the humerus. As illustrated in FIG. 9B, the marker 679 may be a divot on the distal surface 686 of the articular body 656. However, the marker 679 may be printed or otherwise visually indicated on the distal surface 686 or elsewhere on the articular body 656.

The articular body 656 may include a channel 684 disposed between the articular surface 672 and the distal surface 686. The channel 684 may be formed in a surface, for example a side peripheral or circumferential surface. As explained above, the articular portion 652 may include a locking member 602 to ensure mechanical fixation between the articular body 656 and the fracture stem 140. The locking member 602 may be constructed of a resilient material, such as a resilient metal. The locking member 602 may include any of the features of the locking members 253, 302, 502, 502A described above.

As shown in FIGS. 9A-9B, the locking member 602 may be disposed about the articular body 656, for example within the channel 684. The locking member 602 may reversibly secure the articular body 656 against the fracture stem 140 with a simple impaction load by an impactor and mallet but without the use of any other tools for assembling the articular body 656 to the fracture stem 140. When the articular portion 652 is coupled to the fracture stem 140, the locking member 602 is deflected such that an outer periphery of the locking member 602 is disposed within the stem channel 625 and the inner periphery of the locking member 602 is disposed within the articular body channel 684.

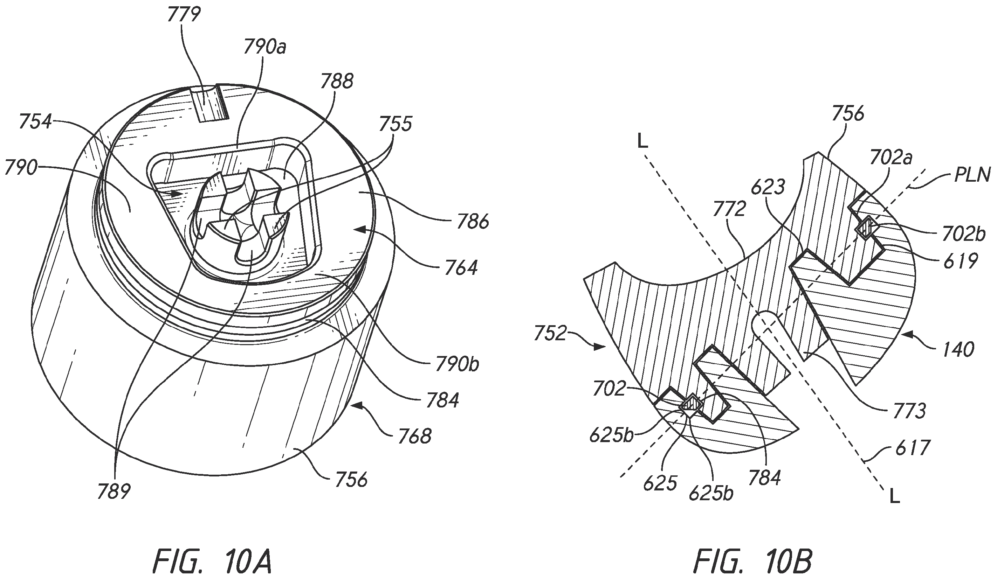

FIG. 10A illustrates another articular portion 752 that is compatible with the fracture stem 140 or one or more other humeral anchors in a variation of the kit 100. The articular portion 752 may include any of the features described above with respect to the articular portions 261, 352, 452, 652. In some embodiments, the articular portion 752 may be an articular assembly, e.g., a polymeric articular body 756 and a locking member 702. In other embodiments, the articular portion 752 may be a single-piece, e.g. a polymeric articular body 756.

As shown in FIG. 10A, the articular body 756 includes a first or distal portion 764 and a second or proximal portion 768. The articular body 756 has an articular surface 772 disposed in the second portion 768. The first portion 764 of the articular body 756 includes a distal surface 786 configured to be disposed in the cavity 619 of the fracture stem 140 (see FIG. 9A). For example, the first portion 764 of the articular body 756 may include a recess 790 extending proximally from the distal surface 786. The shape of the recess 790 may correspond to the raised portion 623 in the proximal face of the fracture stem 140 (see FIG. 9A). The recess 790 may include any features of the recess 690 described above. The articular body 756 may also include a marker 779 to provide an indication of anatomical direction for implantation. The marker 779 may include any of the features of markers 679, 279.

The first portion 764 of the articular body 756 may also include a counter-loaded or deflectable portion 754 projecting distally from a distal face of the articular body 756, for example from the distal facing surface 788 within the recess 790. As illustrated, the deflectable portion 754 has a cylindrical profile, but in other configurations, the deflectable portion 754 may have a frusto-conical profile or other profile. The deflectable portion 754 may be compressible toward the central longitudinal axis L upon insertion into the hole 617 of the fracture stem 140 (see FIG. 10B). The deflectable portion 754 may include any of the features of the deflectable portion 254. For example, the deflectable portion 754 may include at least two sections 755, for example three sections or four sections, cantilevered from a central portion of the articular body 756. The deflectable portion 754 may also include a compression slot 789 disposed between each of the at least two sections 755. For example, as shown in FIG. 10A, the deflectable portion 754 may include four sections 755 separated by intersecting compression slots 756. The compression slot(s) 756 enable the deflectable portion 754 to be compressed in a direction transverse to the longitudinal axis L or compressed in an annular direction as the deflectable portion 754 is inserted into the hole 617.

The articular body 756 may include a channel 784 disposed between the articular surface 772 and the distal surface 786. The channel 784 may be formed in a surface, for example a side peripheral or circumferential surface. The articular portion 752 may include a locking member 702 to ensure mechanical fixation between the articular body 756 and the fracture stem 140. The locking member 702 may be constructed of a resilient material, such as a resilient metal. The locking member 702 may include any of the features of the locking members 253, 302, 502, 502A, 602 described above.

As shown in FIG. 10B, the locking member 702 may be disposed about the articular body 756, for example within the channel 784. The locking member 702 may reversibly secure the articular body 756 against the fracture stem 140 with a simple impaction load by an impactor and mallet but without the use of any other tools for assembling the articular body 756 to the fracture stem 140. When the articular portion 752 is coupled to the fracture stem 140, the locking member 702 is deflected such that an outer periphery of the locking member 702 is disposed within the stem channel 625 and the inner periphery is disposed within the articular body channel 784.

The locking member 702 may include a proximal edge 702a and a distal edge 702b. The distal edge 702b may be disposed at an angle relative to the proximal edge 702a (see FIG. 10B). As the articular portion 752 is inserted into the fracture stem 140, the distal edge 702b of the locking member 702 is advanced toward the distal edge 625b of the channel 625. After impaction, the locking member 702 elastically recovers such that the proximal edge 702a of the locking member 702 contacts the proximal edge 625a of the channel 625.

When the humeral implant is fully assembled, the locking member 702 may be disposed along a plane PLN distal of the proximal face of the fracture stem 140. The PLN may extend transversely through the deflectable portion 752. The locking member 702 may be disposed between the distal surface 786 of the articular body 756 and the distal end 773 of the articular body 756. This provides a compact arrangement along the axis L. This arrangement allows the connection of the deflectable portion 752 and a coupler of an anatomic articular component to be made close to or at the proximal end of the fracture stem 140.

In a variation of the deflectable portion 752, a continuous projection without slots 789 can be provided. For example, a blind hole can be formed in a projection having a tapered outer profile and an enclosed interior, similar to the blind hole 298 of the projection of the embodiment of FIG. 3F.

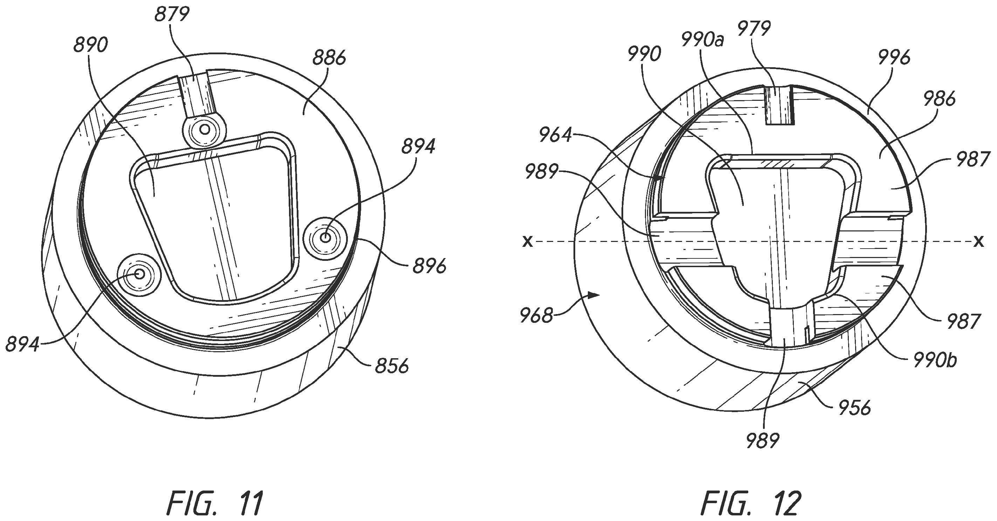

FIGS. 11-12 show other engagement features that may be used in connection with any of the above-identified articular portions to couple the articular portion with the fracture stem 140 or other bone anchor.

FIG. 11 illustrates an articular body 856 having a distal surface 886. The articular body 856 may include a recess 890 extending proximally from the distal surface 886. The shape of the recess 890 may correspond to the raised portion 623 in the proximal face of the fracture stem 140 (see FIG. 9A). The recess 890 may include any features of the recess 690 described above. The articular body 856 may also include a marker 879 including any of the features of markers 679, 279.

The articular body 856 may include one or more deformable projections 894 extending from the distal surface 886. The one or more deformable projections 894 may correspond to one or more corresponding recesses in a proximal face of the bone anchor. The one or more deformable projections 894 are compressed upon insertion into the one or more corresponding recesses. The one or more deformable projections 894 may comprise a deformable material, for example a deformable polymeric material such as UHMWPE. The one or more deformable projections 894 may include a continuous periphery without any gaps, slots, or other discontinuities. At least one deformable projection 894 may be positioned off-center, displaced from the center of the articular body 856. For example, each of the one or more deformable projections 894 may be disposed radially between the recess 890 and an outer periphery 896 of the distal surface 886. The one or more deformable projections 894 may also facilitate rotational alignment between the articular portion 852 and the bone anchor. Any of deformable projections 894 may engage the fracture stem 140, for example by a press fit. Although the illustrated articular body 856 includes three deformable projections 894, a fewer or greater number of deformable projections 894 may be possible.

FIG. 12 illustrates an articular body 956 having a distal surface 986. The articular body 956 includes a first or distal portion 964 and a second or proximal portion 968. The articular body 956 has an articular surface disposed in the second portion 968. The first portion 964 of the articular body 956 includes a distal surface 986 configured to be disposed in the cavity 619 of the fracture stem 140 (see FIG. 9A).

The articular body 956 may include a recess 990 extending proximally from the distal surface 986. The shape of the recess 990 may correspond to the raised portion 623 in the proximal face of the fracture stem 140 (see FIG. 9A). The recess 990 may include any features of the recess 690 described above. For example, the recess 990 may have a straight portion 990a on a lateral side thereof and a curved portion 990b on a medial side thereof. The articular body 956 may also include a marker 979 having any of the features of markers 679, 279.

The entire distal portion 964 may be compressed upon insertion into the cavity 619 of the fracture stem 140 to form a press-fit connection. The distal portion 964 may be separated into at least two sections 987, for example three sections or four sections, by compression slots 989. For example, as shown in FIG. 12, the distal portion 964 may include three sections 987 separated by compression slots 956 extending from the outer periphery 996 to the recess 990. The distal portion 964 may include a first section 987 along the lateral side or the straight portion 990a of the recess 990 and at least one section 987, for example two sections 987, along the medial side or the curved portion 990b of the recess 990. The distal portion 964 may include two compression slots 989 aligned along a transverse axis X. The distal portion may include at least one additional compression slot 989 on an axis perpendicular to the transverse axis X. The compression slots 989 enable the distal portion 964 to be compressed in an annular direction as the distal portion 964 is inserted into the cavity 619 of the fracture stem 140.

The present invention may also relate to the following embodiments:
Embodiment 1. An articular component configured to be coupled with a bone anchor, the articular component comprising:
   an articular body having a first end and a second end;
   an articular surface disposed on or adjacent to the first end;
   a bone anchor interface disposed between the first end and the second end of the articular body, the bone anchor interface comprising:
      a locking member configured to secure the articular component to the bone anchor; and
      a deflectable portion disposed at the second end of the articular body, the deflectable portion configured to be circumferentially deflected by a surface of a humeral anchor to provide a load directed toward the first end of the articular body from a second end of the articular body when deflected.
Embodiment 2. The articular component of Embodiment 1, wherein the bone anchor interface comprises a channel formed in a circumferential surface of the articular body. Embodiment 3.The articular component of Embodiment 2, wherein the locking member is disposed in the channel.
Embodiment 4. The articular component of any one of the preceding Embodiments, wherein the deflectable portion comprises at least two sections cantilevered from a central portion of the articular body to the second end of the articular body.
Embodiment 5. The articular component of Embodiment 4, further comprising a compression slot disposed between each of the at least two sections.
Embodiment 6. The articular component of any one of the preceding Embodiments, wherein the deflectable portion comprises a tapered surface disposed on an outer circumference thereof.
Embodiment7. The articular component of any one of the preceding Embodiments, wherein the articular body comprises a transverse surface configured to overlay a rim of the bone anchor when the locking member is engaged with the bone anchor.
Embodiment 8. The articular component of any one of the preceding Embodiments, further comprising a rotation control zone disposed at a periphery of the articular body between the first end and the second end.
Embodiment 9. The articular component of Embodiment8, wherein the rotation control zone comprises a projection disposed in a first direction and a recess disposed in a second direction.
Embodiment 10. The articular component of Embodiment 9, wherein the first direction is transverse to the second direction.
Embodiment 11. The articular component of Embodiment 9 or 10, wherein the projection is a first projection and further comprising a second projection disposed opposite the first projection.
Embodiment 12. The articular component of any one of Embodiments 9 to 11, wherein the recess is a first recess and further comprising a second recess disposed opposite the first recess.
Embodiment 13. The articular component of any one of the preceding Embodiments, wherein the deflectable portion is disposed between the locking member and the second end of the articular body.
Embodiment 14. A kit comprising:
   the articular component of any one of the preceding Embodiments; and
   a bone anchor having a bone anchor recess formed therein, the bone anchor recess extending from a first end, a bone engagement outer surface extending from the first end to a second end opposite the first end, the bone anchor recess having a first peripheral portion adjacent to the first end configured to engage the locking member of the articular component and a second peripheral portion between the first peripheral portion and the second end, the second peripheral portion configured to engage the deflectable portion.
Embodiment 15. The kit of Embodiment 14, further comprising a tray having a first end and a second end configured to engage the bone anchor recess of the bone anchor at least at the second peripheral portion, wherein the first end of the tray comprises a tray recess formed therein, the tray recess having a first peripheral portion adjacent to the first end configured to engage the locking member of the articular component and a second peripheral portion between the first peripheral portion and the second end, the second peripheral portion configured to engage the deflectable portion.
Embodiment 16. A humeral assembly, comprising:
   a humeral anchor configured to be anchored in a bone, the humeral anchor comprising a first end, a second end, and a recess extending between the first end and the second end, the recess being accessible from the first end of the humeral anchor and comprising a first peripheral portion adjacent to the first end and a second peripheral portion adjacent to the first peripheral portion;
   an articular assembly configured to be inserted into the recess to be secured to the humeral anchor therein, the articular assembly including an articular body having an articular surface disposed on or adjacent to a first end thereof and a humeral anchor interface disposed between the first end and a second end of the articular body, the humeral anchor interface comprising:
      a channel formed in a circumferential surface of the articular body;
      a locking member disposed in the channel;
      a deflectable projection disposed at the second end of the articular body;
   wherein the deflectable projection is configured to be positioned in the second peripheral portion of the recess and when so positioned to be circumferentially deflected to provide friction loading on a surface of the second peripheral portion to provide a load between the locking member and a surface of the first peripheral portion. Embodiment 17. The humeral assembly of Embodiment 16, wherein the locking member comprises a C-ring.
Embodiment 18. The humeral assembly of Embodiment 16 or 17, wherein the deflectable projection comprises at least two sections cantilevered from a central portion of the articular body to the second end of the articular body.
Embodiment 19. The humeral assembly of Embodiment 18, further comprising a compression slot disposed between each of the at least two sections.
Embodiment 20. The humeral assembly of any one of Embodiments 16 to 18, wherein the deflectable projection comprises a tapered surface disposed on an outer circumference thereof.
Embodiment 21. The humeral assembly of Embodiment 18, wherein the deflectable projection comprises four sections, each comprising a cantilevered from a central portion of the articular body to the second end of the articular body.
Embodiment 22. The humeral assembly of any one of Embodiments 16 to 21, wherein the deflectable projection comprises a blind hole along a centerline of the articular body.
Embodiment 23. The humeral assembly of any one of Embodiments 16 to 22, wherein the deflectable projection is configured to engage a surface surrounding the second peripheral portion before the locking member engages the first peripheral portion of the recess of the humeral anchor.
Embodiment 24. The humeral assembly of any one of Embodiments 16 to 23, wherein the humeral anchor comprises a stemless nucleus.
Embodiment 25. The humeral assembly of any one of Embodiments 16 to 23, wherein the humeral anchor comprises a stemmed anchor.
Embodiment 26. The humeral assembly of any one of Embodiments 16 to 25, wherein the humeral anchor comprises a first portion configured to be inserted into a resected humerus and a second portion configured to be coupled with the first portion, the recess being formed in the second portion.
Embodiment 27. The humeral assembly of Embodiment 26, wherein the second portion comprises a tray configured to engage the articular assembly.
Embodiment 28. The humeral assembly of any one of Embodiments 16 to 27, wherein at least one of a projection and a recess disposed in the humeral anchor interface is configured to form an interference fit with a projection or a recess formed in the first peripheral portion of the recess of the bone anchor.
Embodiment 29. The humeral assembly of any one of Embodiments 16 to 28, wherein the deflectable projection is disposed between the locking member and the second end of the articular body.
Embodiment 30. An articular assembly configured to be coupled with a bone anchor, comprising:
   an articular body having a first end and a second end;
   an articular surface disposed on or adjacent to the first end;
   a bone anchor interface disposed between the first end and the second end of the articular body, the bone anchor interface comprising:
      a channel formed in a circumferential surface of the articular body;
      a locking member disposed in the channel;
      a deflectable portion disposed at the second end of the articular body, the deflectable portion being configured to be circumferentially deflected by a surface of the bone anchor whereby a load is directed toward the first end of the articular body from a second end of the articular body.
Embodiment 31. The articular assembly of Embodiment 30, wherein the deflectable portion comprises at least two sections cantilevered from a central portion of the articular body to the second end of the articular body.
Embodiment 32. The articular assembly of Embodiment 31, further comprising a compression slot disposed between each of the at least two sections.
Embodiment 33. The articular assembly of any one of Embodiments 30 to 32, wherein the deflectable portion comprises a tapered surface disposed on an outer circumference thereof.
Embodiment 34. The articular assembly of any one of Embodiments 30 to 33, wherein the articular body comprises a transverse surface disposed between the channel and the first end of the articular body, the transverse surface configured to overlay a rim of the bone anchor when the locking member is engaged with the bone anchor.
Embodiment 35. The articular assembly of any one of Embodiments 30 to 34, further comprising a rotation control zone disposed at a periphery of the articular body between the first end and the second end.
Embodiment 36. The articular assembly of Embodiment 35, wherein the rotation control zone comprises a projection disposed in a first direction and a recess disposed in a second direction.
Embodiment 37. The articular assembly of Embodiment 36, wherein the first direction is transverse to the second direction.
Embodiment 38. The articular assembly of any one of Embodiments 35 to 37, wherein the projection is a first projection and further comprising a second projection disposed opposite the first projection.
Embodiment 39. The articular assembly of any one of Embodiments 35 to 38, wherein the recess is a first recess and further comprising a second recess disposed opposite the first recess.
Embodiment 40. The articular assembly of any one of Embodiments 35 to 39, wherein the deflectable projection is disposed between the locking member and the second end of the articular body.
Embodiment 41. A kit comprising:
   the articular assembly of any one of Embodiments 30 to 40;
   a bone anchor having a bone anchor recess formed therein, the bone anchor recess extending from a first end, a bone engagement outer surface extending from the first end to a second end opposite the first end, the bone anchor recess having a first peripheral portion adjacent to the first end configured to engage the locking member of the articular assembly and a second peripheral portion between the first peripheral portion and the second end, the second peripheral portion configured to engage the deflectable portion; and
   a tray having a first end and a second end configured to engage the bone anchor recess of the bone anchor at least at the second peripheral portion,
   where the first end of the tray comprises a tray recess formed therein, the tray recess having a first peripheral portion adjacent to the first end configured to engage the locking member of the articular assembly and a second peripheral portion between the first peripheral portion and the second end, the second peripheral portion configured to engage the deflectable portion.
Embodiment 42. A method, comprising:
   positioning a bone anchor in an end of a long bone of a patient;
   rotationally aligning a first rotational alignment feature of an articular assembly with a second rotational alignment feature disposed in a recess, the recess being formed in the bone anchor or in a tray coupled with the bone anchor disposed at the end of the long bone;
   advancing the articular assembly until a counter-load projection of the articular assembly is disposed within a tapered surface of the recess;
   further advancing the articular assembly until a locking member of the articular assembly is deflected within a channel formed in a central portion of an articular body of the articular assembly and the counter-load projection is deflected by the tapered surface of the recess;
   further advancing the articular assembly until the locking member of the articular assembly is aligned with a channel disposed about the recess in the bone anchor to permit the locking member to span a gap between the channel in the articular body and the channel disposed about the recess;
   wherein when the counter-load projection is deflected by the tapered surface of the recess, the counter-load projection reduces, minimizes or eliminates motion of the articular assembly relative to the bone anchor after the locking member spans the gap between the channel in the articular body and the channel disposed about the recess.
Embodiment 43. The method of Embodiment 42, comprising rotationally aligning a projection of the articular assembly with a concavity formed in a circumference surrounding the recess.
Embodiment 44. The method of Embodiment 42 or 43, wherein further advancing comprises engaging tapered outer surfaces of the counter-load projection with a tapered surface of the recess to move sections of the counter-load projection toward each other across compression slots thereof.
Embodiment 45. The method of any one of Embodiments 42 to 44, further comprising directly engaging the articular assembly with a recess formed in the bone anchor. Embodiment 46. The method of any one of Embodiments 42 to 45, further comprising engaging a tray with a bone anchor recess, the tray having a tray recess configured to directly engage the articular assembly.
Embodiment 47. A shoulder joint prosthesis assembly, comprising:
   a bone anchor comprising a metallic body having a bone engaging side to be placed against bone and an assembly side opposite the bone engaging side, the assembly side comprising a recess disposed about a mounting area and a channel disposed peripherally about the mounting area, the channel including a first retention surface;
   an articular assembly, comprising:
      a polymeric body extending along a central insertion axis between a first portion configured to be inserted into the mounting area and a second portion opposite the first portion, the second portion including an articular surface; and
      a locking member comprising:
         an arcuate member disposed at least partially about the first portion, the arcuate member comprising a second retention surface; and
         a resilient body having a first end extending from the arcuate member and a second end disposed radially inward of the arcuate member;
   wherein the second retention surface is disposed at an angle (α) to a plane disposed perpendicular to the central insertion axis, such that a first portion of the second retention surface is disposed farther from the articular surface in the direction of the central insertion axis than is the first retention surface when the articular assembly is engaged with the bone anchor and a second portion of the second retention surface disposed closer to the articular surface in the direction of the central insertion axis than is the first retention surface when the articular assembly is engaged with the bone anchor.
Embodiment 48. The shoulder joint prosthesis assembly of Embodiment 47, wherein the angle (α) is greater than 0 degrees.
Embodiment 49. The shoulder joint prosthesis assembly of Embodiment 47 or 48, wherein the first portion of the polymeric body comprises a recess configured to receive the resilient body in only one position.
Embodiment 50. The shoulder joint prosthesis assembly of any one of Embodiments 47 to 49, wherein the resilient body stores strain energy upon application of a deflection force to deflect the locking member away from a centered position and releases the strain energy to return the locking member toward the centered position upon removal of the deflection force.
Embodiment 51. The shoulder joint prosthesis assembly of any one of Embodiments 47 to 50, wherein the second end of the resilient body is coupled to the polymeric body. Embodiment 52. The shoulder joint prosthesis of any one of Embodiments 47 to 51, wherein an inner periphery or an outer periphery of the arcuate member comprises stiffness reduction feature.
Embodiment 53. The shoulder joint prosthesis assembly of any one of Embodiments 47 to 52, wherein the resilient body comprises a radial portion extending radially inward from a first end of the arcuate member and an arcuate portion extending from the radial portion.
Embodiment 54. The shoulder joint prosthesis assembly of Embodiment 53, wherein the arcuate portion is concentric with the arcuate member.
Embodiment 55. The shoulder joint prosthesis assembly of Embodiment 53 or 54, wherein the arcuate member defines a gap between the first end of the arcuate member and the second end of the arcuate member.
Embodiment 56. The shoulder joint prosthesis assembly of Embodiment 55, the arcuate portion overlaps the gap.
Embodiment 57. The shoulder joint prosthesis assembly of any one of Embodiments 47 to 56, wherein the locking member further comprises a second arcuate member disposed at least partially about the first portion.
Embodiment 58. The shoulder joint prosthesis assembly of Embodiment 57, wherein the resilient body extends between the arcuate member and the second arcuate member.
Embodiment 59. The shoulder joint prosthesis of any one of Embodiments 47 to 58, wherein the resilient body comprises a base at the second end of the resilient body, the base configured to be secured in the recess of the first portion of the polymeric body.
Embodiment 60. The shoulder joint prosthesis of Embodiment 59, wherein the resilient body comprises a first deflectable member extending from the base to the first end of the arcuate member at another end.
Embodiment 61. The shoulder joint prosthesis assembly of Embodiment 60, wherein the resilient body further comprises a second deflectable member extending from the base to the second arcuate member.
Embodiment 62. The shoulder joint prosthesis assembly of any one of Embodiments 47 to 61, wherein the locking member comprises at least one additional resilient body having a first end extending from the arcuate member and a second end disposed radially inward of the arcuate member, the at least one additional resilient body being circumferentially spaced apart from the resilient body.
Embodiment 63. The shoulder joint prosthesis assembly of Embodiment 62, wherein the at least one additional resilient body comprises two resilient bodies.
Embodiment 64. A shoulder joint prosthesis assembly, comprising:
   a bone anchor comprising a metallic body having a bone engaging side to be placed against bone and an assembly side opposite the bone engaging side, the assembly side comprising a recess disposed about a mounting area and a channel disposed peripherally about the mounting area, the channel including a first retention surface;
   an articular assembly, comprising:
      a polymeric body extending along a central insertion axis between a first portion configured to be inserted into the mounting area and a second portion opposite the first portion, the second portion including an articular surface; and
      a locking member comprising:
         an arcuate member disposed about the first portion; and
         a resilient body having a first end extending from the arcuate member and at a second end disposed radially inward of the arcuate member, the resilient body configured to center the arcuate member relative to the central insertion axis.
Embodiment 65. The shoulder joint prosthesis assembly of Embodiment 64, further comprising a locator body at the second end of the resilient body, the locator body configured to engage the polymeric body in a pre-defined position and/or orientation.
Embodiment 66. The shoulder joint prosthesis assembly of Embodiment 65, wherein the polymeric body comprises a recess and the locator body is further configured to be disposed in the recess in only one rotation position relative to the central insertion axis.
Embodiment 67. The shoulder joint prosthesis assembly of any one of Embodiments 64 to 66, wherein the second end of the resilient body is coupled to the polymeric body.
Embodiment 68. The shoulder joint prosthesis assembly of any one of Embodiments 64 to 67, wherein the arcuate member comprises a second retention surface disposed along a plane disposed at an acute angle to a plane normal to the central insertion axis, the first retention surface being disposed between inner and outer ends of the second retention surface when the locking member is engaged with the channel of the bone anchor.
Embodiment 69. The shoulder joint prosthesis assembly of any one of Embodiments 64 to 68, wherein the resilient body stores strain energy upon application of a deflection force to deflect the locking member away from a centered position and releases the strain energy to return the locking member toward the centered position upon removal of the deflection force.
Embodiment 70. The shoulder joint prosthesis of any one of Embodiments 64 to 69, wherein an inner periphery or an outer periphery of the arcuate member comprises a stiffness reduction feature.
Embodiment 71. A shoulder joint prosthesis assembly, comprising:
   a polymeric body extending along a central insertion axis between a first portion configured to be inserted into a bone anchor and a second portion opposite the first portion, the second portion including an articular surface; and
   a locking member comprising an arcuate member disposed about the first portion and a locator body having a first end coupled to the arcuate member and a second end coupled to the polymeric body, the locator body configured to be received in a recess of the polymeric body in a pre-defined orientation and position.
Embodiment 72. The shoulder joint prosthesis assembly of Embodiment 71, wherein the pre-defined orientation is one rotation position relative to the central insertion axis. Embodiment 73. The shoulder joint prosthesis assembly of Embodiment 71, wherein the predefined position is a centered position.
Embodiment 74. The shoulder joint prosthesis assembly of any one of Embodiments 71 to 73, wherein the locator body is configured to store strain energy upon application of a deflection force to deflect the first end of the locking member away from the centered position and releases the strain energy to return the locking member toward the centered position upon removal of the deflection force.
Embodiment 75. The shoulder joint prosthesis assembly of any one of Embodiments 71 to 74, further comprising:
   a bone anchor comprising a metallic body having a bone engaging side to be placed against bone and an assembly side opposite the bone engaging side, the assembly side comprising a recess disposed about a mounting area configured to receive the first portion of the polymeric body of the shoulder joint assembly and a groove disposed peripherally about the mounting area configured to receive the arcuate member of the connection component.
Embodiment 76. The shoulder joint prosthesis assembly of any one of Embodiments 71 to 75, wherein the locator body is coupled to the polymeric body.
Embodiment 77. The shoulder joint prosthesis of any one of Embodiments 71 to 76, wherein an inner periphery or an outer periphery of the arcuate member comprises a stiffness reduction feature.

### Terminology

Although certain embodiments have been described herein, the implants and methods described herein can interchangeably use any articular component, as the context may dictate.

As used herein, the relative terms "proximal" and "distal" shall be defined from the perspective of the implant. Thus, proximal refers to the direction of the articular component and distal refers to the direction of an anchor component, such as a stem of a humeral anchor or a thread or porous surface or other anchoring structure of a stemless anchor when the implant is assembled.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments.

The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. In addition, the articles "a," "an," and "the" as used in this application and the appended claims are to be construed to mean "one or more" or "at least one" unless specified otherwise.

The ranges disclosed herein also encompass any and all overlap, sub-ranges, and combinations thereof. Language such as "up to," "at least," "greater than," "less than," "between," and the like includes the number recited. Numbers preceded by a term such as "about" or "approximately" include the recited numbers and should be interpreted based on the circumstances (e.g., as accurate as reasonably possible under the circumstances, for example ±5%, ±10%, ±15%, etc.). For example, "about 1" includes "1." Phrases preceded by a term such as "substantially," "generally," and the like include the recited phrase and should be interpreted based on the circumstances (e.g., as much as reasonably possible under the circumstances). For example, "substantially spherical" includes "spherical." Unless stated otherwise, all measurements are at standard conditions including temperature and pressure.

As used herein, a phrase referring to "at least one of" a list of items refers to any combination of those items, including single members. As an example, "at least one of: A, B, or C" is intended to cover: A, B, C, A and B, A and C, B and C, and A, B, and C. Conjunctive language such as the phrase "at least one of X, Y and Z," unless specifically stated otherwise, is otherwise understood with the context as used in general to convey that an item, term, etc. may be at least one of X, Y or Z. Thus, such conjunctive language is not generally intended to imply that certain embodiments require at least one of X, at least one of Y and at least one of Z to each be present.

Although certain embodiments and examples have been described herein, it should be emphasized that many variations and modifications may be made to the humeral head assembly shown and described in the present disclosure, the elements of which are to be understood as being differently combined and/or modified to form still further embodiments or acceptable examples. All such modifications and variations are intended to be included herein within the scope of this disclosure. A wide variety of designs and approaches are possible. No feature, structure, or step disclosed herein is essential or indispensable.

Some embodiments have been described in connection with the accompanying drawings. However, it should be understood that the figures are not drawn to scale. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

For purposes of this disclosure, certain aspects, advantages, and novel features are described herein. It is to be understood that not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Moreover, while illustrative embodiments have been described herein, it will be understood by those skilled in the art that the scope of the inventions extends beyond the specifically disclosed embodiments to any and all embodiments having equivalent elements, modifications, omissions, combinations or sub-combinations of the specific features and aspects of the embodiments (e.g., of aspects across various embodiments), adaptations and/or alterations, and uses of the inventions as would be appreciated by those in the art based on the present disclosure. The limitations in the claims are to be interpreted broadly based on the language employed in the claims and not limited to the examples described in the present specification or during the prosecution of the application, which examples are to be construed as non-exclusive. Further, the actions of the disclosed processes and methods may be modified in any manner, including by reordering actions and/or inserting additional actions and/or deleting actions. It is intended, therefore, that the specification and examples be considered as illustrative only, with a true scope and spirit being indicated by the claims and their full scope of equivalents.

Any methods disclosed herein need not be performed in the order recited. The methods disclosed herein include certain actions taken by a practitioner; however, they can also include any third-party instruction of those actions, either expressly or by implication. For example, actions such as "coupling a glenoid guide with the glenoid rim" include "instructing coupling of a glenoid guide with a glenoid rim."

## Claims

1. A kit, comprising:
a bone anchor (203) including:
a first recess (231) extending distally from a proximal face (239) into a proximal portion (207),
a second recess (232) extending distally from the first recess (231), and
an inner periphery (233) disposed about the first recess (231);
an articular component (261) configured to be coupled to the bone anchor, the articular component including:
an articular body (280) having a body portion (287), an articular surface (293), a rotational control zone, and a transverse surface (286), and
a bone anchor interface (288) having a deflectable portion (254), the deflectable portion (254) projecting distally from the rotational control zone (285) and configured to be compressible in a direction transverse to a longitudinal axis (L) of the articular body (280) or in an annular direction;
wherein the inner periphery (233) includes one or more alignment features configured to interface with corresponding features of the rotational control zone (285).

2. The kit of claim 1, wherein the bone anchor interface (288) includes a channel (284) formed in a circumferential surface of the articular body (280) and is configured to to dispose therein a locking member (253) for locking the articular component to the bone anchor (203).

3. The kit of claim 2, wherein the locking member (253) is a locking ring configured to be seated in the channel (284) formed in a circumferential surface of the articular body (280) and to be engaged in a channel (244) extending circumferentially along the inner periphery (233) of the bone anchor (203).

4. The kit of any one of the previous claims, wherein the rotational control zone (285) includes a first projection (252) and a second projection (252) circumferentially spaced apart from the first projection.

5. The kit of claim 4, wherein the bone anchor (203) includes a plurality of concave locking features (243) spaced apart from one another along the inner periphery (233).

6. The kit of any one of the previous claims, wherein the rotational control zone (285) includes a recess (251) in the form of a concave slot.

7. The kit of any one of the previous claims, wherein the deflectable portion (254) includes at least two sections (255) cantilevered from a central portion of the articular body (280) and includes a compression slot (256) disposed between each of the at least two sections (255).

8. The kit of any one of claims 1 to 6, wherein the deflectable portion (254) includes a projection (297) having a blind hole (298) extending proximally through the projection (297).

9. The kit of any one of the previous claims, wherein a volume of the first recess (231) is larger than a volume of the second recess (232).

10. The kit of any one of the previous claims, wherein, when the articular component (261) is assembled with the bone anchor (203), the deflectable portion (254) is deflected to cause a load to be applied in a direction opposite to a direction in which the articular component (261) was advanced relative to the bone anchor (203).

11. The kit of any one of the previous claims, wherein the transverse surface (286) of the articular body (280) is configured to overlay the proximal face (239) of the bone anchor (203) when the articular component (261) is assembled with the bone anchor (203).

12. The kit of any one of the previous claims, further comprising:
an anatomic articular body (164); and
a coupler (168) configured to mechanically connect the anatomic articular body (164) to the bone anchor (203).

13. The kit of claim 12, wherein the coupler includes a proximal extension (163A) configured to connect to the anatomic articular body (164), a distal extension (163B), and a middle portion (162) disposed between the proximal extension (163A) and the distal extension (163B).

14. The kit of any one of the previous claims, wherein a proximal portion of the body portion (287) is angled with respect to the transverse surface (286).

15. The kit of any one of the previous claims, wherein the bone anchor includes a stem (283).
